# EUROPEAN PATENT APPLICATION

(11) **EP 4 458 375 A1**
(43) Date of publication of application: **06.11.2024**
(21) Application number: 22916131.0
(22) Date of filing: 27.12.2022
(51) Int. Cl.: A61K 45/00, A61K 31/404, A61K 31/439, A61K 31/4412, A61K 31/4439, A61K 31/47, A61K 31/496, A61K 31/506, A61K 31/517, A61K 31/53, A61K 31/5377, A61K 39/395, A61P 9/00, C07D 209/34, C07D 213/81, C07D 215/22, C07D 239/94, C07D 403/06, C07D 513/04, C12Q 1/6869, G01N 33/53

(54) **PHARMACEUTICAL COMPOSITION FOR TREATING AND/OR PREVENTING ARTERIAL ANEURYSM, METHOD FOR ASSISTING DIAGNOSIS OF ARTERIAL ANEURYSM, AND METHOD FOR ASSESSING THERAPEUTIC DRUG FOR ARTERIAL ANEURYSM**

(30) Priority: 27.12.2021 WO PCT/JP2021/048561
(71) Applicant: Nakatomi, Hirofumi, Wako-shi, Saitama 351-0198 (JP)
(72) Inventor: Nakatomi, Hirofumi, Wako-shi, Saitama 351-0198 (JP)
(74) Representative: Altmann Stößel Dick Patentanwälte PartG mbB
(86) International application number: PCT/JP2022/048341
(87) International publication number: WO 2023/127909

(57) **Abstract**

A therapeutic drug useful for the treatment of aneurysms is provided. The pharmaceutical composition for the treatment and/or prevention of aneurysms comprises at least one of the following drugs as an active ingredient(s):
i) drugs targeting platelet-derived growth factor receptor β (PDGFRβ);
ii) drugs that inhibit the function of desmoyokin (AHNAK); and
iii) drugs that inhibit signaling enhanced by mutations in PDGFRβ or AHNAK.

## Description

### Technical Field

The present invention relates to a pharmaceutical composition for the treatment and/or prevention of aneurysms, a method for assisting in the diagnosis to predict the effect of treating and/or preventing aneurysms using specific drugs, and a method for evaluating the therapeutic effects of drugs on aneurysms *in vitro.*

### Background Art

Aneurysms are morphologically abnormal sac-like structures where blood vessels bulge outward, occurring at arterial branch points or arterial trunks. Aneurysms are classified based on their location as thoracic aortic aneurysms, abdominal aortic aneurysms, visceral artery aneurysms, peripheral artery aneurysms, cerebral aneurysms, coronary artery aneurysms, etc. Among these, cerebral aneurysms are a major cause of subarachnoid hemorrhage and the most destructive hemorrhagic stroke. The natural history of giant or large thrombosed fusiform basilar artery aneurysms is extremely poor, with 91% of symptomatic cases leading to death within an average of 5.5 years due to rupture, brainstem infarction, or brainstem dysfunction, leaving severe sequelae in the remaining 9%. There is no established treatment for cerebral aneurysms; current surgical interventions include surgery or endovascular catheter treatment. The prognosis after these treatments is 65-73% good (independent in daily life), 3-15% poor (requiring assistance), and 20-24% mortality, making it a challenging disease to treat.

To date, the genetic factors of aneurysms are not clear. For cerebral aneurysms, recent studies have shown that somatic mutations in PDGFRβ are associated with the development of intracranial fusiform aneurysms (Non-Patent Literature 1).

### Citation List

### Non-Patent Literature

Non-Patent Literature 1: Karasozen et al. (2019) Somatic PDGFRB Activating Variants in Fusiform Cerebral Aneurysms, Am. J. Human Genet. 104, 968-976

### Summary of Invention

### Technical Problem

There is a strong desire for treatment methods that may substitute for or be used in conjunction with surgical interventions to provide more reliable treatments for aneurysms. The present invention aims to provide therapeutic drugs useful for treating aneurysms. Furthermore, the invention aims to provide methods for assisting diagnosis capable of predicting the treatment effects of drugs in subjects with aneurysms. Additionally, it aims to provide methods for evaluating the treatment effects of drugs on aneurysms *in vitro.*

### Solution to Problem

To solve the above issues, the inventor has conducted intensive research and identified specific somatic gene mutations frequently occurring in aneurysm tissues. The inventor also found that drugs targeting specific genes that mutate in aneurysms or drugs that inhibit the function of these genes, or drugs that inhibit the signal transduction enhanced by these mutations, are useful for treating aneurysms.

The present application discloses embodiments described below.
(1) A pharmaceutical composition for the treatment and/or prevention of an aneurysm comprising at least one of the following drugs as an active ingredient(s):
   i) drugs targeting platelet-derived growth factor receptor β (PDGFRβ);
   ii) drugs that inhibit the function of desmoyokin (AHNAK); and
   iii) drugs that inhibit signaling enhanced by mutations in PDGFRβ or AHNAK.
(2) The pharmaceutical composition of (1), wherein the drug(s) targets PDGFRβ.
(3) The pharmaceutical composition of (2), wherein the drug(s) inhibits the activity of tyrosine kinase.
(4) The pharmaceutical composition of (3), wherein the drug(s) is at least one tyrosine kinase inhibitor(s) selected from the group consisting of sunitinib, axitinib, dasatinib, gefitinib, erlotinib, lapatinib, pazopanib, vandetanib, afatinib, regorafenib, cabozantinib, osimertinib, dacomitinib, quizartinib, capmatinib, tepotinib, imatinib, sorafenib, nilotinib, crizotinib, ponatinib, ceritinib, nintedanib, lorlatinib, entrectinib, and their pharmaceutically acceptable salts, solvates, and derivatives.
(5) The pharmaceutical composition of (4), wherein the drug(s) is at least one selected from axitinib, dasatinib, and their pharmaceutically acceptable salts, solvates, and derivatives.
(6) The pharmaceutical composition of (5), wherein the drug(s) is at least one selected from dasatinib and its pharmaceutically acceptable salts, solvates, and derivatives.
(7) The pharmaceutical composition of (1), wherein the drug(s) is the agent(s) targeting AHNAK.
(8) The pharmaceutical composition of (7), wherein the drug(s) is at least one selected from fibroblast growth factor receptor (FGFR) inhibitors, protein kinase C (PKC) inhibitors, and phosphatidylinositol 3-kinase (PI3K) inhibitors.
(9) The pharmaceutical composition of any one of (1) to (8), wherein the aneurysm is a cerebral aneurysm.
(10) A method for assisting in the diagnosis of an aneurysm, comprising the following steps:
   - Detecting mutations in at least one of the PDGFRβ gene and AHNAK gene in a sample collected from a subject with an aneurysm; and
   - Predicting the treatment and/or preventive effect of the pharmaceutical composition described in Claim 1 on the subject.
(11) The method described in (10), wherein mutations in the PDGFRβ gene in the sample are detected to predict the treatment and/or preventive effect of a tyrosine kinase inhibitor on the subject.
(12) The method described in (11), wherein the PDGFRβ gene mutation corresponds to at least one mutation selected from the group consisting of p.559_562del, p.563_564del, p.Y562N, p.Y562S, and p.Y562D in the amino acid sequence represented by SEQ ID NO: 2.
(13) The method described in (12), wherein the PDGFRβ gene mutation corresponds to the p.559_562del mutation in the amino acid sequence represented by SEQ ID NO: 2.
(14) The method described in (10), wherein mutations in the AHNAK gene in the sample are detected to predict the treatment and/or preventive effect of an FGF-R inhibitor, PKC inhibitor, or PI3K inhibitor on the subject.
(15) The method described in (14), wherein the AHNAK gene mutation corresponds to at least one mutation selected from the group consisting of p.D1083Sfs6, p.A2114T, p.G3819A, p.N3827S, p.E3850K, p.A4046V, p.D4701E, and p.H5817Rfs20 in the amino acid sequence represented by SEQ ID NO: 16.
(16) The method described in (15), wherein the AHNAK gene mutation corresponds to the p.G3819A mutation in the amino acid sequence represented by SEQ ID NO: 16.
(17) The method described in any one of (10) to (16), wherein the sample is serum or plasma.
(18) A method for determining treatment effect of a drug on an aneurysm, comprising the following steps b) to d):
   b) contacting human cells with the drug *in vitro*;
   c) measuring the amount of phosphorylated extracellular signal-regulated kinase (p-ERK) and/or phosphorylated tyrosine in the cells from step b;
   d) predicting the treatment effect of the drug on an aneurysm from the measurement value(s) in step c).
(19) A method for determining treatment effect of a drug on an aneurysm, comprising the following steps a') to d'):
   a') preparing human cells into which a mutated PDGFRβ gene has been introduced;
   b') contacting the cells with the drug *in vitro*;
   c') measuring the amount(s) of phosphorylated extracellular signal-regulated kinase (p-ERK) and/or phosphorylated tyrosine in the cells from step b');
   d') predicting the treatment effect of the drug on an aneurysm from the measurement value(s) in step c').
(20) The method described in (18) or (19), wherein the PDGFRβ gene mutation(s) corresponds to at least one mutation(s) selected from the group consisting of p.559_562del, p.563_564del, p.Y562N, p.Y562S, and p.Y562D in the amino acid sequence represented by SEQ ID NO: 2.

### Advantageous Effects of Invention

According to the present invention, it is possible to provide a pharmaceutical useful for the treatment and/or prevention of aneurysms. Additionally, according to the present invention, it is possible to provide a method for assisting diagnosis capable of predicting the treatment/preventive effects of pharmaceuticals in subjects with aneurysms. Furthermore, the invention allows for providing an evaluation method that may assess the treatment/preventive effects of drugs on aneurysms *in vitro.*

### Brief Description of Drawings

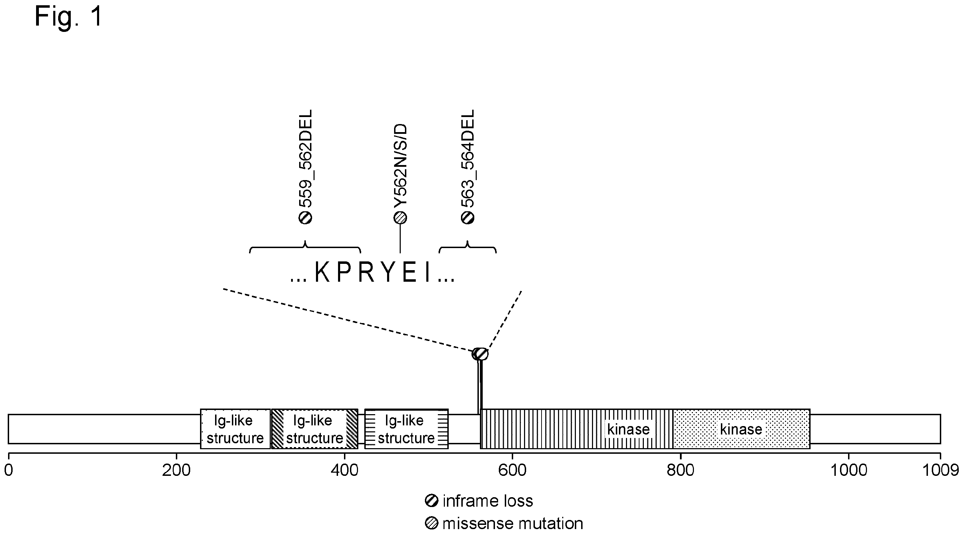

[Figure 1] Figure 1 shows a schematic diagram of the location and content of the most frequent somatic mutations in the PDGFRβ gene in cerebral aneurysm tissue. The novel mutations shown in this schematic diagram were identified by whole exome sequencing.

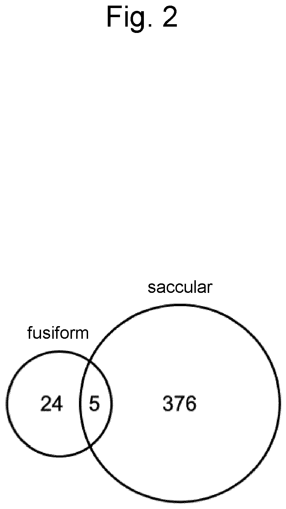

[Figure 2] Figure 2 is a Venn diagram showing the relationship between mutations found in saccular aneurysms (sac) and those found in fusiform aneurysms (fusiform) for nonsynonymous somatic mutations affecting 405 genes detected in whole exome sequencing of tissue samples from 69 patients with cerebral aneurysms. 381 mutations were found in saccular aneurysms and 29 in fusiform aneurysms, with 5 mutations found in both.

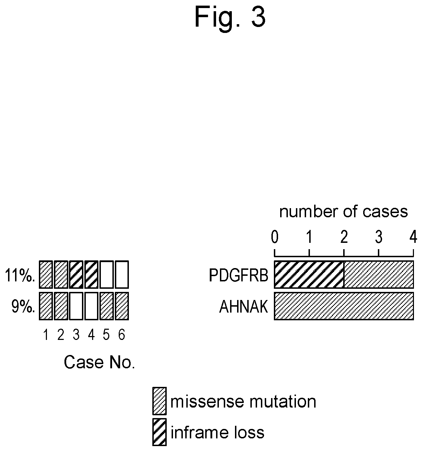

[Figure 3] Figure 3 shows the case-specific distribution of the two somatic mutation genes that were mutated in multiple cases. 6 of the 45 cases had mutations in one of these two genes. The bars on the right indicate the number of cases with each gene mutation, in order of frequency from top to bottom; PDGFRβ mutations were observed in 4 cases and AHNAK mutations in 4 cases.

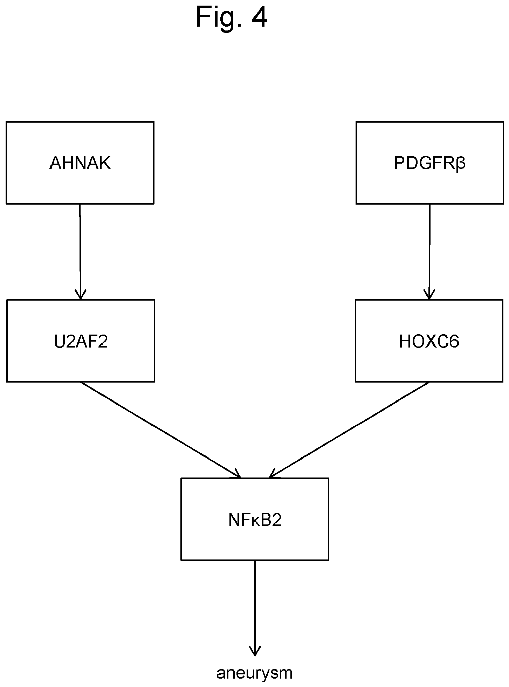

[Figure 4] Figure 4 is a diagram using Inaugural Pathway Analysis (IPA) showing the correlation between somatic mutation genes and aneurysms. PDGFRβ is associated with signaling pathways through HOXC6 and NFκB2, respectively, and AHNAK is associated with signaling pathways through U2AF2 and NFκB2, respectively.

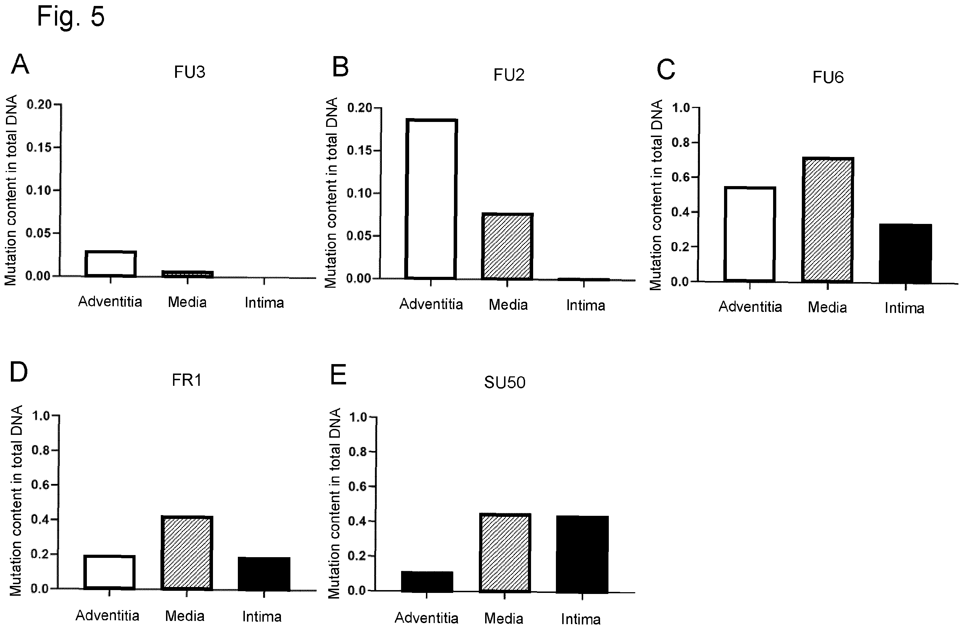

[Figure 5] Figure 5 shows the distribution of PDGFRβ in the cerebral vascular layer of patients with cerebral aneurysms in five cases. (A) Case FU3, showing a small fusiform dilation, had the highest PDGFRβ mutation in the outer membrane layer (adventitia) and very little in the inner membrane layer (intima). (B) Case FU2 with the second smallest size 3-lobulated fusiform aneurysm showed that mutations were most abundant in the adventitial layer, followed by the medial layer (media). (C) Case FU6 with 7-10 mm circumferential thick fusiform aneurysms of moderate size showed that mutations were most abundant in the media, followed by the adventitia, then the intima. (D) Aneurysm in case FR1 with a ruptured fusiform aneurysm of 11 mm shows that PDGFRβ mutations were higher in the medial, adventitial, and intimal layers, in that order. (E) In case SU50 with a 22 mm unruptured saccular giant aneurysm, PDGFRβ mutations were found in the tunica media and intimal layers to the same extent.

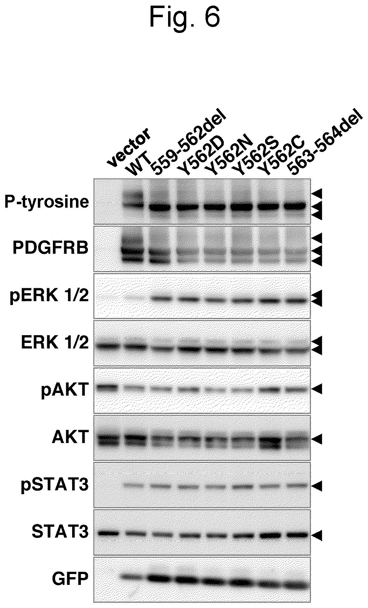

[Figure 6] Figure 6 is a Western blot showing the phosphorylation states of PDGFRβ, ERK1/2, AKT, and STAT3 in HEK293T cells overexpressing each PDGFRβ mutant related to aneurysms. Phosphorylation of PDGFRβ was detected in all mutants tested; phosphorylation of ERK1/2 was detected in all mutants except wild type; phosphorylation of ERK1/2 was highest in the K559_Y562 deletion mutant; phosphorylation of STATS was increased in all mutants; no effect on AKT phosphorylation was observed. In the figure, GFP was detected as a control to confirm the expression of the mutations, and β-actin (AKT) was detected as a control to normalize the intensity of each band in the Western blot.

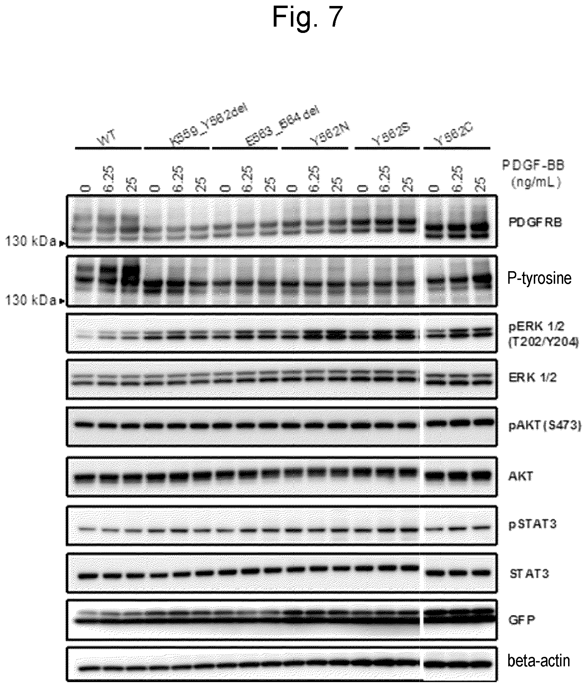

[Figure 7] Figure 7 is a Western blot showing the effect of PDGF-BB administration on HEK293T cells overexpressing each PDGFRβ mutant related to aneurysms. p.559_562del mutant slightly suppressed the phosphorylation of PDGFRβ, but no inhibition of phosphorylation of the other Y562 variants was observed. A PDGF-BB dose-dependent increase in phosphorylation of ERK was observed in all PDGFRβ mutants. In the figure, GFP was detected as a control to confirm the expression of the mutation and β-actin as a control to normalize the intensity of each band in Western blot.

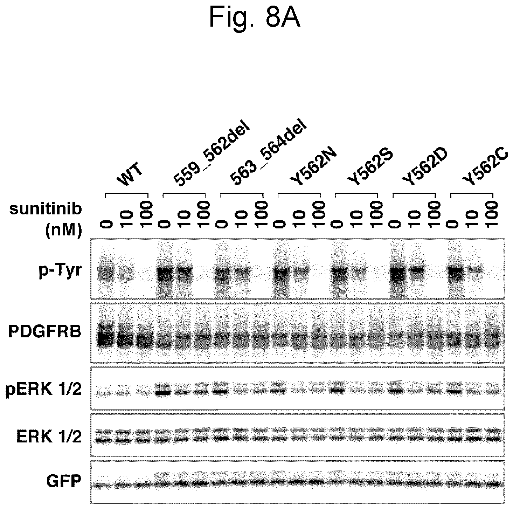

[Figure 8A] Figure 8A is a Western blot showing the effect of the tyrosine kinase inhibitor sunitinib treatment on HEK293T cells overexpressing each PDGFRβ mutant related to aneurysms. Phosphorylation of tyrosine with PDGFRβ was suppressed by sunitinib. three Y562 mutants, p.559_562del, and p.563_564del, ERK phosphorylation was suppressed by PDGFRβ inactivation.

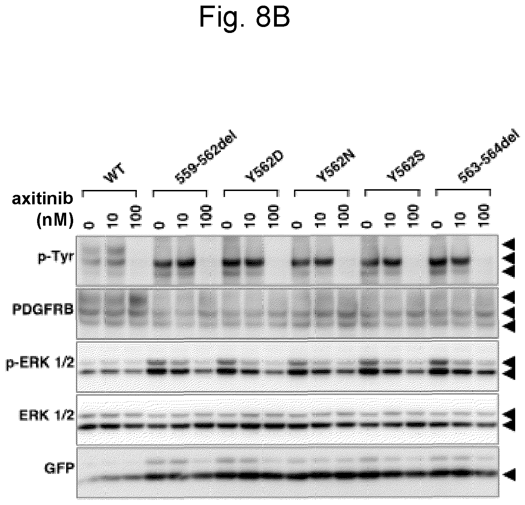

[Figure 8B] Figure 8B is a Western blot photograph showing the effect of axitinib treatment, a tyrosine kinase inhibitor, in HEK293T cells overexpressing each PDGFRβ mutant associated with aneurysms. Phosphorylation of tyrosine with PDGFRβ was suppressed by axitinib. Three Y 562 mutants, p.559_562del, and p.563_564del mutants, ERK phosphorylation was suppressed by PDGFRβ inactivation.

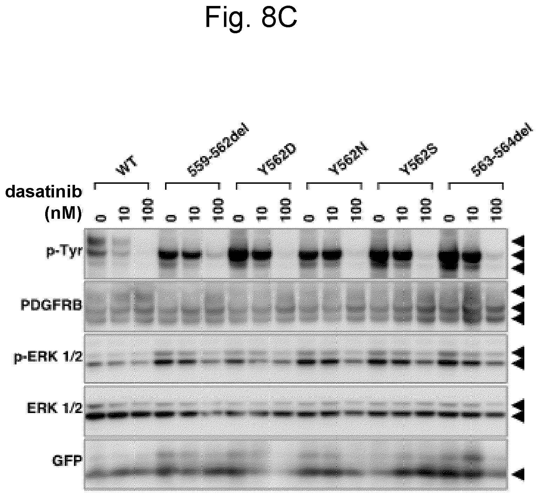

[Figure 8C] Figure 8C is a Western blot photograph showing the effect of dasatinib treatment, a tyrosine kinase inhibitor, in HEK293T cells overexpressing each PDGFRβ mutant associated with aneurysms. Phosphorylation of tyrosine with PDGFRβ was suppressed by dasatinib. Three Y562 mutants, p.559_562del, and p.563_564del, ERK phosphorylation was suppressed by PDGFRβ inactivation.

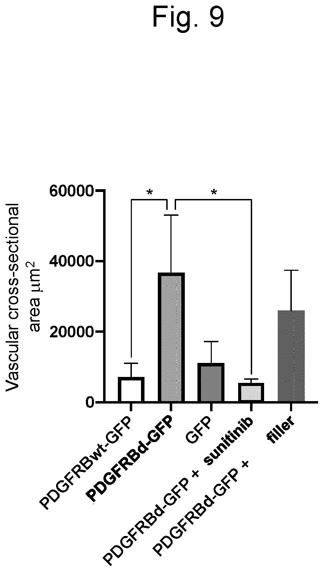

[Figure 9] Figure 9 shows the results of the measurement of vascular diameter of basilar arteries in a mouse model of cerebral aneurysms. Mice were transfected with the PDGFRβwt (wild-type) or PDGFRβd (p. 559_562del) gene along with the GFP gene; mice transfected with GFP alone were used as controls; expression of PDGFRβ wild-type GFP and GFP alone did not change the vascular cross-sectional area over time; vascular cross-sectional area was greatly enlarged in mice expressing the above mutants compared to wild-type (*P<0.05). The addition of sunitinib suppressed the vascular enlargement.

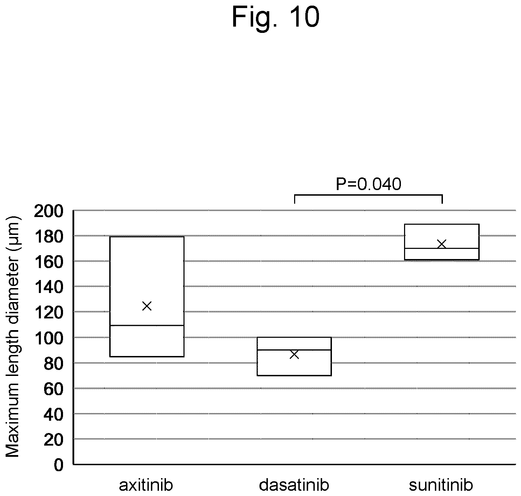

[Figure 10] Figure 10 shows the distribution of the maximum length diameter of basilar artery in mice introduced PDGFRβ mutation under each drug administration condition. The p-values in the figure were calculated by Bonferroni using one-way ANOVA.

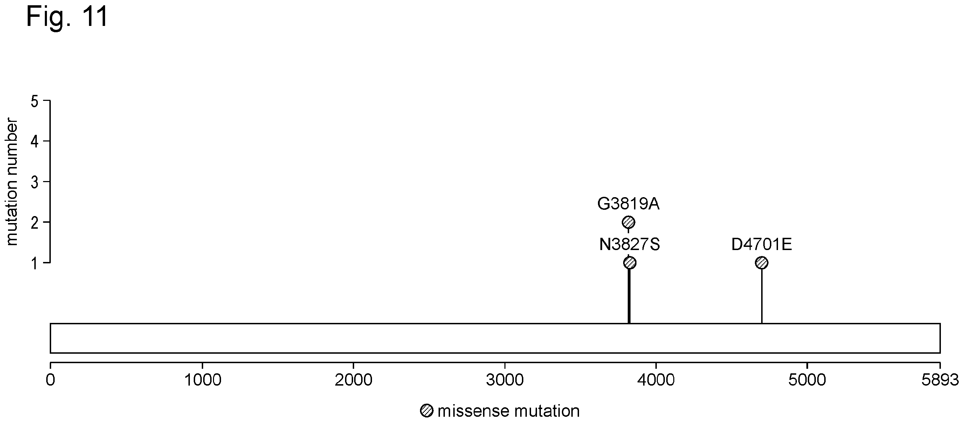

[Figure 11] Figure 11 shows the position and content of somatic mutations in the AHNAK gene observed by whole-exome sequencing.

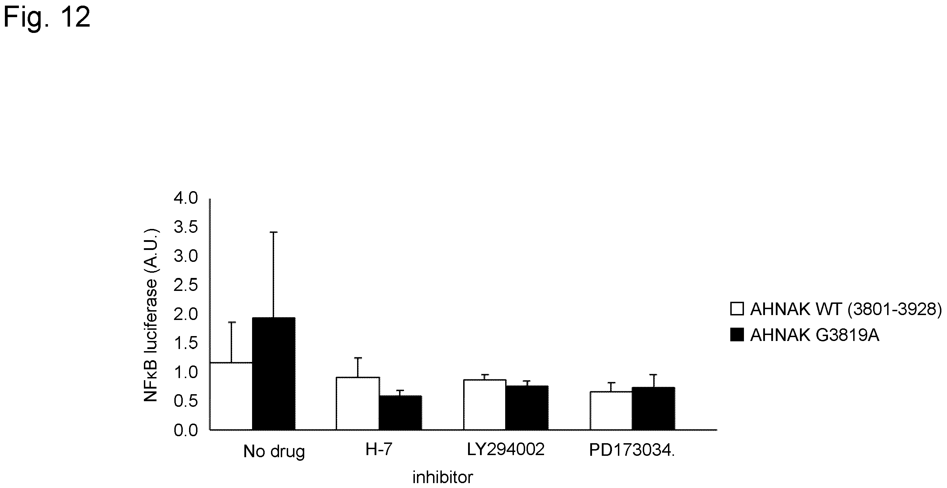

[Figure 12] Figure 12 shows the results of NFκB luciferase reporter assay in HEK293T cells transfected with wild-type AHNAK and p.G3819A mutant AHNAK in the presence of various inhibitors.

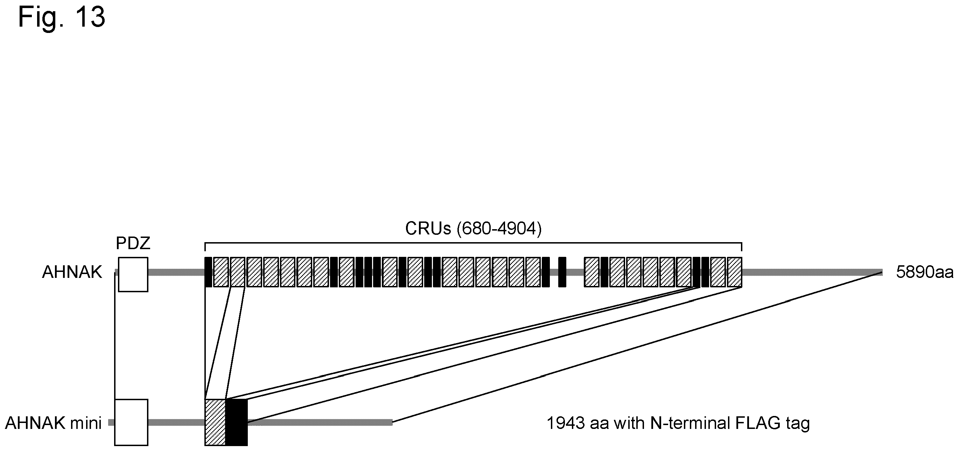

[Figure 13] Figure 13 is a schematic diagram showing the structure of AHNAKmini.

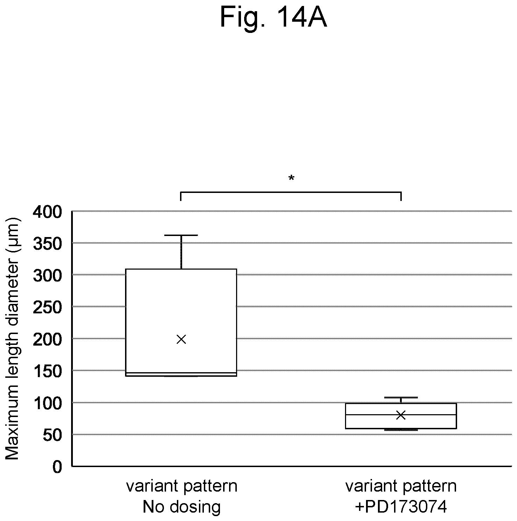

[Figure 14A] Figure14A is a graph showing the maximum length diameters of the internal carotid artery and middle cerebral artery with and without PD173074 in a mouse model of cerebral aneurysm introduced AHNAK mutation. Error bars in the figure indicate standard error. The "*" in the figure indicates that p<0.05 in the Student's t-test of equal variances with no correspondence.

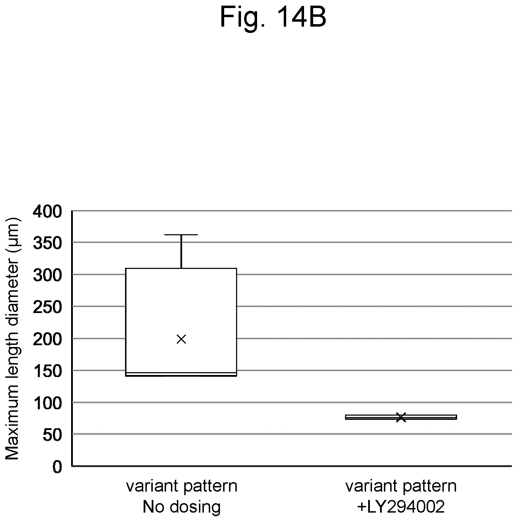

[Figure 14B] Figure 14B shows carotid and middle cerebral arteries with and without administration of LY294002 in a mouse model of cerebral aneurysm transduced with the AHNAK mutation. Error bars in the figure indicate standard error.

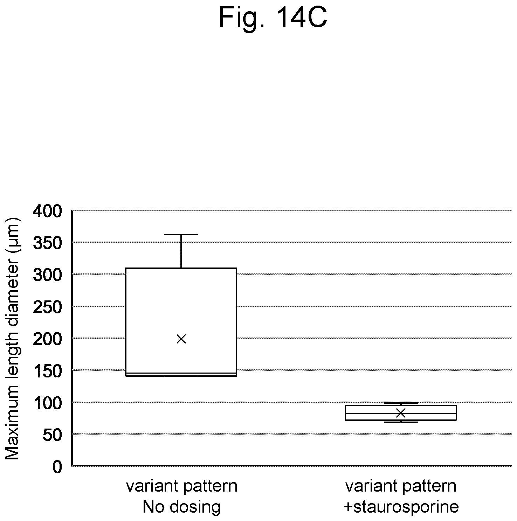

[Figure 14C] Figure 14C shows carotid and middle cerebral arteries with and without staurosporine in a mouse model of cerebral aneurysm transfected with an AHNAK mutation. Error bars in the figure indicate standard error.

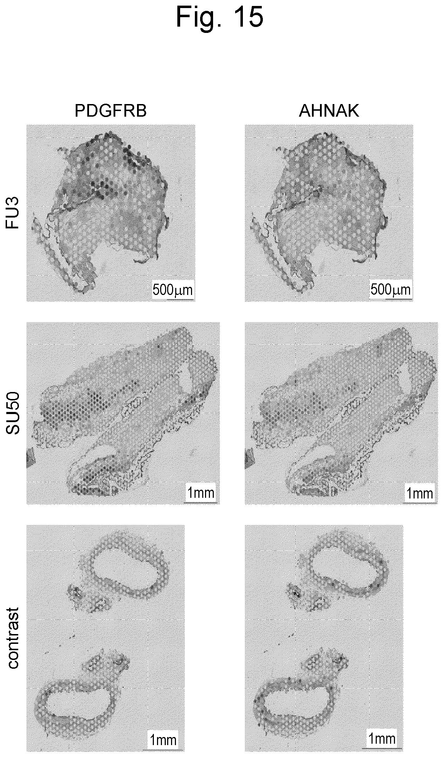

[Figure 15] Figure 15 shows the results of mRNA expression analysis of PDGFRβ and AHNAK in frozen cerebrovascular tissue sections of two CA tissues from cerebral aneurysm (CA) patients and one control using the Visium spatial gene expression analysis system. The darker reddish areas are the areas with higher mRNA expression. The expression of both genes was upregulated in a wide range of CA tissues compared to the control.

### Description of Embodiments

### <Definitions>

Unless otherwise specified, the compounds described herein encompass all possible geometric isomers and optical isomers. A "pharmaceutically acceptable salt" of a compound herein refers, unless otherwise specified, to a salt formed with cations such as sodium, potassium, calcium, magnesium, or substituted or unsubstituted ammonium ions, or with anions of inorganic acids such as hydrochloric acid, hydrobromic acid, sulfuric acid, nitric acid, carbonic acid, or phosphoric acid, or with anions of organic acids such as formic acid, acetic acid, maleic acid, fumaric acid, benzoic acid, ascorbic acid, lactic acid, succinic acid, bis-methylene salicylic acid, methanesulfonic acid, ethanedisulfonic acid, propionic acid, tartaric acid, malic acid, salicylic acid, citric acid, gluconic acid, aspartic acid, stearic acid, palmitic acid, itaconic acid, glycolic acid, p-aminobenzoic acid, glutamic acid, benzenesulfonic acid, cyclohexylsulfamic acid, methanesulfonic acid, ethanesulfonic acid, isethionic acid, p-toluenesulfonic acid, naphthalenesulfonic acid, mesylate, or tosylate. The term "solvate" of a compound includes, unless otherwise specified, solvates with water or organic solvents such as lower alcohols (e.g., methanol, ethanol, or 2-propanol (isopropyl alcohol) with 1-6 carbon atoms), higher alcohols (e.g., 1-heptanol or 1-octanol with 7 or more carbon atoms), dimethyl sulfoxide (DMSO), acetic acid, ethanolamine, or ethyl acetate. The term "derivative" of a compound includes, unless otherwise specified, a compound in which one or more hydrogen atoms (H) are substituted with halogen (fluorine, chlorine, bromine, or iodine), cyano, nitro, hydroxyl, substituted or unsubstituted C1-C5 alkyl, substituted or unsubstituted C2-C5 alkenyl, substituted or unsubstituted C2-C5 alkynyl, substituted or unsubstituted C3-C6 cycloalkyl, substituted or unsubstituted C3-C6 cycloalkenyl, substituted or unsubstituted C3-C6 cycloalkynyl, substituted or unsubstituted 3-6 membered heterocycloalkyl, substituted or unsubstituted C7-C11 cycloalkylalkyl, substituted or unsubstituted 3-6 membered heterocycloalkyl-C1-C5 alkyl, substituted or unsubstituted C6-C15 aryl, substituted or unsubstituted C7-C20 arylalkyl, substituted or unsubstituted 5-15 membered heteroaryl, substituted or unsubstituted 5-15 membered heteroaryl-C1-C5 alkyl, substituted or unsubstituted C1-C6 alkoxy, substituted or unsubstituted C3-C6 cycloalkoxy, substituted or unsubstituted 3-6 membered heterocycloalkoxy, substituted or unsubstituted C6-C15 aryloxy, substituted or unsubstituted C7-C20 arylalkyloxy, substituted or unsubstituted 5-15 membered heteroaryloxy, substituted or unsubstituted 5-15 membered heteroaryl-C1-C5 alkyloxy, substituted or unsubstituted C1-C6 alkoxycarbonyl, substituted or unsubstituted C3-C6 cycloalkoxycarbonyl, substituted or unsubstituted aminocarbonyl (carbamoyl), substituted or unsubstituted C1-C20 acyl, substituted or unsubstituted C1-C20 acyloxy, substituted or unsubstituted amino, substituted or unsubstituted sulfonamide, substituted or unsubstituted carbonyl diimino, carboxyl, and oxo, and their pharmaceutically acceptable salts and solvates. In this context, "substituted or unsubstituted" substituents refer to groups where part of the substituent is further substituted with any of the aforementioned substituents. Furthermore, "derivatives" in the present invention include, unless otherwise specified, compounds in which one or more nitrogen atoms (N) or oxygen atoms (O) are bonded to any of the aforementioned substituents, and their pharmaceutically acceptable salts and solvates.

The notation "A to B" (where A and B are numerical values) herein refers to "A or more and B or less" unless otherwise specified. As used herein, "%" refers to "weight %" unless otherwise specified or contradictory.

As used herein, "aneurysm" refers to a balloon-like bulging section of an artery. "Cerebral aneurysm (CA)" refers to an aneurysm occurring in the cerebral arteries. Unruptured cerebral aneurysms are found in 2-6% of adults without symptoms. Cerebral aneurysms frequently occur at branch points of the cerebral basal arteries, including the middle cerebral artery, internal carotid artery, anterior communicating artery, and basilar artery. The size of cerebral aneurysms varies from 2mm to 25mm in diameter, with most being less than 10mm. Many are asymptomatic but may cause headaches by compressing nerves. Giant cerebral aneurysms pose a high risk of subarachnoid hemorrhage upon rupture, with a rupture rate of 33% per year for aneurysms larger than 25mm. Cerebral aneurysms are classified based on their shape into saccular cerebral aneurysms (SCA) and fusiform cerebral aneurysms (FCA), with SCA posing a higher rupture risk.

As used herein, "tyrosine kinase inhibitor (TKI)" refers to enzyme inhibitors that block the action of one or more tyrosine kinases, which phosphorylate tyrosine in proteins. Tyrosine kinases are crucial enzymes involved in signal transduction related to cell growth and differentiation, and abnormal activation may lead to cell carcinogenesis. Commercial tyrosine kinase inhibitors are primarily used as anti-cancer agents.

As used herein, "platelet-derived growth factor receptor β (PDGFRβ)" refers to a tyrosine kinase protein that belongs to the family of receptor tyrosine kinases (RTKs) involved in various signal transduction cascades during embryonic development. It is expressed in a limited number of cell types, including vascular smooth muscle cells and pericytes, playing a crucial role in vascular progenitor cell signaling. The PDGFRβ gene is also known as an oncogene. Mutations in the PDGFRβ gene are hypothesized to enhance tyrosine kinase activity in aneurysm formation. Human PDGFRβ has an amino acid sequence represented by SEQ ID NO: 2 or a sequence with at least 80%, 85%, 90%, 95%, 96%, 97%, 98%, or 99% identity to the sequence represented by SEQ ID NO: 2 and possesses tyrosine kinase activity. The PDGFRβ gene encoding PDGFRβ has a nucleotide sequence encoding a protein having an amino acid sequence represented by SEQ ID NO: 2 or a sequence with at least 80%, 85%, 90%, 95%, 96%, 97%, 98%, or 99% identity to the sequence represented by SEQ ID NO: 2. The nucleotide sequence of PDGFRβ gene may have an exon sequence represented by SEQ ID NO: 1 (NCBI Reference sequence NM_002609) or a sequence with at least 80%, 85%, 90%, 95%, 96%, 97%, 98%, or 99% identity to the sequence represented by SEQ ID NO: 1, for example, provided that the nucleotide sequence encodes the aforementioned protein.

Proteins phosphorylated by PDGFRβ include, for example, extracellular signal-regulated kinase (ERK), protein kinase B (AKT), and STAT3.

In this specification, "desmoyokin (AHNAK)" refers to a large structural protein associated with muscle fiber physiology and a tumor suppressor factor mediated by transforming growth factor-β (TGF-β/Smad signaling. Human AHNAK has an amino acid sequence represented by SEQ ID NO: 16 or a sequence with at least 80%, 85%, 90%, 95%, 96%, 97%, 98%, or 99% identity to the sequence represented by SEQ ID NO: 16. The AHNAK gene encoding human AHNAK has an exon sequence represented by SEQ ID NO: 15 (NCBI Reference Sequence: NM_001620) or a sequence with at least 80%, 85%, 90%, 95%, 96%, 97%, 98%, or 99% identity to the sequence represented by SEQ ID NO: 15.

In this specification, "whole exome sequencing (WES)" refers to sequencing the exonic regions of the entire genome. "Targeted deep sequencing (DS)" refers to sequencing specific regions of the sample DNA at high depth, allowing for the identification of minor somatic mutations within tissues.

In this specification, a "sample" refers to a sample taken from a subject with an aneurysm, including fluids such as blood (whole blood, plasma, serum, and blood cells), lymph fluid, urine, saliva, sweat, semen, tissue fluid, body cavity fluid, cerebrospinal fluid, and tissues such as blood vessel walls and cerebral tissue.

In this specification, gene mutations are described as follows. At the DNA level, base substitutions are denoted as "c.1684T>A", where "c." stands for coding DNA. "c.1684T>A" indicates a mutation where thymine at position 1684 is replaced by adenine. Base deletions are denoted as "c.1675_1686del", indicating a deletion of bases from positions 1675 to 1686. At the protein level, missense mutations are denoted as "p.Y562N", where "p." stands for protein. "p.Y562N" indicates a mutation where tyrosine at position 562 is replaced by asparagine. Amino acid deletions are denoted as "p.559_562del", indicating a deletion of amino acids from positions 559 to 562. Frameshift mutations are denoted as "p.D1083Sfs*6", indicating a frameshift starting with serine at position 1083 and ending with a stop codon at the sixth position in the new reading frame.

### <Pharmaceutical Compositions>

The pharmaceutical compositions in the present invention are for the treatment and/or prevention of aneurysms, comprising at least one of the following drugs as an active ingredient:
i) drugs targeting platelet-derived growth factor receptor β (PDGFRβ);
ii) drugs that inhibit the function of desmoyokin (AHNAK); and
iii) drugs that inhibit signaling enhanced by mutations in PDGFRβ or AHNAK.

It allows for non-invasive treatment and/or prevention of aneurysms. Hereafter, any of the drugs in i) to iii) may be referred to as "PDGFRβ-related drug(s)" or "AHNAK-related drug(s)".

The inventor obtained basilar artery tissues and blood samples from 69 cases of cerebral aneurysm patients. Through Whole Exome Sequencing (WES) and Deep Sequencing (DS), they discovered that multiple genes, including Platelet-Derived Growth Factor Receptor β (*PDGFRβ*) and desmoyokin (*AHNAK*), frequently exhibited mutations in both saccular cerebral aneurysms (SCA) and fusiform cerebral aneurysms (FCA). The genes with the most mutations across patients were PDGFRβ gene and AHNAK gene.

Additionally, DS was performed on cerebral aneurysm tissue samples from 68 cases to analyze the PDGFRβ and AHNAK genes. For *PDGFRβ*, WES detected four types of mutations in four cases, and DS identified one additional mutation in one case, totaling five mutations in five cases (refer to "Reference Example 1" for details). Furthermore, it was confirmed that PDGFRβ with mutations was expressed in the vascular walls of the basilar artery in cerebral aneurysm patients.

Figure 1 provides a schematic representation of the positions and types of the five mutations identified in *PDGFRβ* through WES and DS. Specifically, the mutations include two missense mutations (p.Y562N, p.Y562S, p.Y562D), a four-amino acid in-frame deletion in exon 12 (p.559_562del), and a two-amino acid in-frame deletion (p.563_564del).

For the AHNAK gene, WES detected three types of mutations in four cases. Figure 11 shows the positions of these three mutations. DS further detected five types of mutations in eight additional cases. The mutations identified in the AHNAK gene include six missense mutations (p.A2114T, p.G3819A, p.N3827S, p.E3850K, p.A4046V, and p.D4701E) and two frameshift mutations (p.D1083Sfs6 and p.H5817Rfs).

Aneurysms are broadly classified into SCA and FCA. As a trend, cases with FCA more frequently exhibited mutations in PDGFRβ, whereas cases with SCA often had mutations in AHNAK. Interestingly, within SCA, cases with giant saccular aneurysms frequently exhibited mutations in PDGFRβ.

Using Inaugural Pathway Analysis (IPA) to analyze the signaling pathways involving PDGFRβ and AHNAK in aneurysm formation revealed that PDGFRβ is associated with signaling pathways mediated by HOXC6 and NFκB2, while AHNAK is linked to signaling pathways mediated by U2AF2 and NFκB2, forming a signaling network for aneurysm formation, as shown in Figure 4. It was found that mutations in both genes may cause excessive signaling, contributing to inflammation leading to aneurysm formation.

In human embryonic kidney cells (HEK293T) transfected with the PDGFRβ gene harboring each of 5 mutations, tyrosine kinase activity was found to be enhanced compared to cells transfected with wild-type *PDGFRβ*. Furthermore, this enhancement was suppressed to the level observed with wild-type *PDGFRβ* upon treatment with tyrosine kinase inhibitors.

The inventor confirmed that infection of mouse cerebral vessels with adeno-associated virus (AAV) carrying PDGFRβ gene mutations promoting cerebral aneurysm formation (specifically, p.559_562del mutation) led to basilar artery dilation, indicating aneurysm formation. Administration of tyrosine kinase inhibitors (sunitinib, dasatinib, axitinib) starting at the time of virus infection and on the seventh day post-infection suppressed this arterial dilation. This demonstrated, for the first time, the preventative and treatment effects of tyrosine kinase inhibitors on cerebral aneurysms.

In human embryonic kidney cells (HEK293T) transfected with AHNAK partial genes harboring mutations (p.G3819A), an increase in the expression of NFκB, an inflammation marker, was observed. It was also found that NFκB expression in these cells could be suppressed by fibroblast growth factor receptor (FGF-R) inhibitors, protein kinase C (PKC) inhibitors, and phosphatidylinositol 3-kinase (PI3K) inhibitors.

Due to the large molecular weight of AHNAK, direct introduction into experimental animals via viral vectors proved challenging. The inventor cloned a gene coding for a region of AHNAK that comprises the mutation (equivalent to p.G3819A) and retains partial function. Infection of mouse cerebral vessels with AAV carrying this AHNAK partial gene (AHNAKmini) led to expansion of maximum diameter of a cerebral blood vessel, indicating aneurysm formation. Furthermore, administration of fibroblast growth factor receptor (FGF-R) inhibitors, protein kinase C (PKC) inhibitors, and phosphatidylinositol 3-kinase (PI3K) inhibitors from the seventh day post-infection suppressed this expansion of the cerebral blood vessel.

Based on the above findings, the inventor has identified the utility of PDGFRβ-related drugs, particularly tyrosine kinase inhibitors, in the treatment and/or prevention of aneurysms. Additionally, the inventor has found that AHNAK-related drugs, specifically fibroblast growth factor receptor (FGF-R) inhibitors, protein kinase C (PKC) inhibitors, and phosphatidylinositol 3-kinase (PI3K) inhibitors, are useful for the treatment and/or prevention of aneurysms.

The pharmaceutical composition in the present invention is used for the treatment and/or prevention of aneurysms. The aneurysms may be symptomatic or asymptomatic and there are no particular restrictions on the size of the aneurysms. This composition is especially suitable for subjects with asymptomatic and small unruptured aneurysms that have traditionally been monitored without aggressive surgical intervention. For preventive use, it may be particularly useful in preventing recurrence in subjects post-surgery, or for high-risk individuals (due to genetic factors, environmental factors, lifestyle habits, etc.) who have not yet developed symptoms.

The "drug(s)" comprised in the pharmaceutical composition in the present invention is not particularly limited provided that it may suppress the inflammatory reactions and/or abnormal cell proliferation that may result from the increased activity due to mutations in PDGFRβ and/or AHNAK.

As PDGFRβ-related drugs, tyrosine kinase inhibitors are preferably used. Such tyrosine kinase inhibitors include sunitinib, axitinib, dasatinib, gefitinib, erlotinib, lapatinib, pazopanib, vandetanib, afatinib, regorafenib, cabozantinib, osimertinib, dacomitinib, quizartinib, capmatinib, tepotinib, imatinib, sorafenib, nilotinib, crizotinib, ponatinib, ceritinib, nintedanib, lorlatinib, entrectinib, and their pharmaceutically acceptable salts, solvates, and derivatives. Among these, axitinib (Formula I) and dasatinib (Formula II), and their pharmaceutically acceptable salts, solvates, and derivatives, which demonstrated high treatment/preventive effects in the aneurysm model mice constructed by the inventor, are preferably used. In particular, dasatinib, and its pharmaceutically acceptable salts, solvates, and derivatives, are preferred.

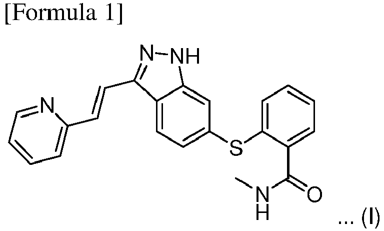

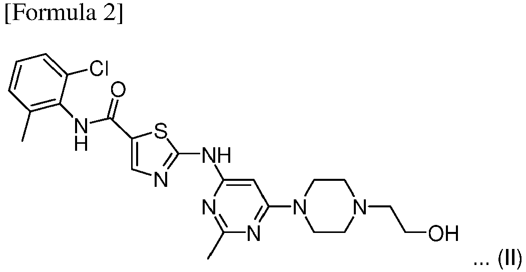

As shown in Figure 4, Inaugural Pathway Analysis (IPA) revealed that mutations in the PDGFRβ gene are involved in inflammatory signaling pathways mediated by HOXC6 and NFκB2. NFκB2 is a transcription factor known to promote the expression of inflammatory cytokines. It also acts to enhance the expression of prostaglandin E1 (PGE1), which has vasodilatory effects. The HOXC6 gene is known as an oncogene expressed in cancers such as prostate cancer, and the HOXC6 protein encoded by this gene is known as a transcription factor. It is thought that mutations in the PDGFRβ gene enhance HOXC6 signaling, which in turn enhances NFκB2 signaling. Therefore, PDGFRβ-related drugs may include inhibitors targeting HOXC6 (e.g., anti-human HOXC6 monoclonal antibodies or their binding fragments that may inhibit HOXC6 transcriptional activity, siRNAs targeting HOXC6 mRNA, anticancer drugs for cancers resulting from abnormal HOXC6 protein activity), and inhibitors targeting NFκB2 (e.g., TPCA1, BOT-64, BMS345541, Amlexanox, SC-514, IMD0354, IKK-16, BAY 11-7082, MG-115, MG-132, Lactacystin, Epoxomicin, Parthenolide, Carfilzomib, MLN-4924, JSH-23, Rolipram, Gallic acid, Anacardic acid, GYY 4137, p-XSC, LY 294002, Wortmannin, Quinacrine, Mesalamine, CV 3988, Flavopiridol, BAY 11-7082, JSH-23, QNZ, SC75741, Pyrrolidinedithiocarbamate ammonium). Specific antibodies or their binding fragments that directly inhibit the tyrosine kinase activity of PDGFRβ may also be used. The PDGFRβ-related drugs are not limited to the above-mentioned drugs provided that they may suppress excessive inflammatory responses and/or abnormal cell proliferation due to PDGFRβ mutations.

The pharmaceutical composition in the present invention may also comprise the AHNAK-related drug(s), in addition to or instead of the PDGFRβ-related drug(s). The choice of drugs may be determined by predicting effective drugs using the method for assisting the diagnosis described later, or based on clinical findings of the aneurysm (e.g., whether it is SCA or FCA).

AHNAK is reported to be involved in signal transduction through phosphorylation of Smad2/3 in the Transforming Growth Factor β (TGFβ)/Smad signaling pathway (Lee I, H., et al., Oncogene, 33(8), 4675-4684 (2014)). Therefore, inhibiting the signal transduction pathways contributing to TGFβ signaling, specifically by targeting FGF-R, PKC and PI3K, was predicted to be effective for the treatment/prevention of aneurysm formation. This was confirmed through research, and thus FGF-R inhibitors, PKC inhibitors, and PI3K inhibitors were validated for their efficacy in treating/preventing aneurysm formation. Consequently, "AHNAK-related drug(s)" include FGF-R inhibitors (e.g., futibatinib, pemigatinib, E7090, sorafenib, ponatinib, infigratinib, nintedanib, pazopanib, PD173074, dovitinib, AZD4547, danusertib, lenvatinib, brivanib, MK-2461, thiazofurin, SSR128129E, R1530, FIIN-3, lucitanib, surufatinib, ODM-203, ASP5878, derazantinib, nintedanib ethanesulfonate, H3B-6527, roblitinib (FGF401), PRN1371, PD166866, fisogatinib, S49076, NSC12, ON123300, SU5402, BLU9931, FIIN-2, zoligratinib, LY2874455, ferulic acid, masitinib, BO-264, SKLB 610, alofanib, gambogic acid, erdafitinib, their pharmaceutically acceptable salts, solvates, and derivatives), PKC inhibitors (e.g., H-7, enzastaurin, GSK690693, fasudil, mitoxantrone, staurosporine, Go 6983, bisindolylmaleimide I, bisindolylmaleimide IX, daphnetin, decarine, A-3, N-desmethyltamoxifen, bisindolylmaleimide VIII, H-1152, HA-100, rottlerin, bisindolylmaleimide IV, VTX-27, valrubicin, ML-7, rebuxostatine, midostaurin, Go6976, chelerythrine, epsilon-V1-2, N-desmethyltamoxifen, hypericin, oncrasin-1, darovasertib, TAS-301, 2-methoxy-1,4-naphthoquinone, quercetin, myricitrin, methyl-hesperidin, their pharmaceutically acceptable salts, solvates, and derivatives), and PI3K inhibitors (e.g., LY294002, idelalisib, copanlisib, alpelisib, duvelisib, buparlisib, dactolisib, leniolisib, nemiralisib, parsaclisib, pictilisib, apitolisib, gedatolisib, omipalisib, desapiens, bimiralisib, samolisib, taselisib, eganelisib, inavolisib, their pharmaceutically acceptable salts, solvates, and derivatives). Here, the FGF-R inhibitors, PKC inhibitors, and PI3K inhibitors are not limited to the compounds listed, and any compound known to inhibit FGF-R, PKC, or PI3K, regardless of its other physiological activities, may be comprised.

As shown in Figure 4, IPA revealed that mutations in the AHNAK gene are involved in inflammatory signaling pathways mediated by U2AF2 and downstream NFκB2. NFκB2 is a transcription factor known to enhance the expression of inflammatory cytokines. It also acts to enhance the expression of prostaglandin E1 (PGE1), which has vasodilatory effects. The U2AF2 gene is known as an oncogene expressed in cancers such as breast cancer, colorectal cancer, lung cancer, and prostate cancer, and the U2AF2 protein is known to form a complex with U2AF1 protein and be involved in splicing. Mutations in the AHNAK gene may enhance interactions mediated by U2AF2 and downstream NFκB2, promoting inflammatory responses and abnormal cell proliferation, potentially leading to aneurysm formation. Therefore, in addition to FGF-R inhibitors, PKC inhibitors, and PI3K inhibitors, the "AHNAK-related drug(s)" may include inhibitors targeting U2AF2 (e.g., anti-human U2AF2 monoclonal antibodies or their binding fragments that reduce binding activity with U2AF1 or reduce splicing activity of the U2AF1/U2AF2 complex, siRNAs targeting U2AF2 mRNA, anticancer drugs for cancers resulting from abnormal U2AF2 protein activity), and inhibitors targeting NFκB2 (e.g., TPCA1, BOT-64, BMS345541, Amlexanox, SC-514, IMD0354, IKK-16, BAY 11-7082, MG-115, MG-132, Lactacystin, Epoxomicin, Parthenolide, Carfilzomib, MLN-4924, JSH-23, Rolipram, Gallic acid, Anacardic acid, GYY 4137, p-XSC, LY 294002, Wortmannin, Quinacrine, Mesalamine, CV 3988, Flavopiridol, BAY 11-7082, JSH-23, QNZ, SC75741, Pyrrolidinedithiocarbamate ammonium, their pharmaceutically acceptable salts, solvates, and derivatives). Alternatively, gene therapy drugs such as siRNA targeting AHNAK genes with or without specific mutations may also be used. The AHNAK-related drugs are not limited to the above-mentioned drugs provided that they may suppress excessive inflammatory responses and/or abnormal cell proliferation due to AHNAK mutations.

The subjects to whom the pharmaceutical composition in the present invention is administered include primates such as humans and chimpanzees, pet animals such as dogs and cats, livestock animals such as cows, horses, sheep, and goats, rodents such as mice and rats, and other mammals such as zoo animals, preferably humans. More preferably, the subject is a human with an aneurysm (also referred to as "aneurysm patient"). Most preferably, the subject is a human with a cerebral aneurysm (also referred to as "cerebral aneurysm patient").

When the subject is a human, it is particularly preferable that the subject has any of the following gene mutations: *PDGFRβ*: mutations represented by p.559_562del, p.Y562N, p.Y562S, p.Y562D, and p.563_564del in the amino acid sequence represented by SEQ ID NO:2; *AHNAK*: mutations represented by p.D1083Sfs6, p.A2114T, p.G3819A, p.N3827S, p.E3850K, p.A4046V, p.D4701E, and p.H5817Rfs20 in the amino acid sequence represented by SEQ ID NO: 16.

The pharmaceutical composition in the present invention comprises PDGFRβ-related drug(s) and/or AHNAK-related drug(s) (hereinafter simply referred to as "drug(s)") as active ingredient(s). Preferably, the pharmaceutical composition in the present invention comprises tyrosine kinase inhibitor(s) as active ingredient(s) for PDGFRβ-related drug(s). Alternatively, the pharmaceutical composition comprises FGF-R inhibitor(s), PKC inhibitor(s), and/or PI3K inhibitor(s) as active ingredient(s) for AHNAK-related drug(s). The content of the drug(s) as active ingredient(s) in the pharmaceutical composition varies depending on several factors such as the efficacy and stability of the drug, the form of the pharmaceutical composition, the type of carrier used, the method of administration, and the condition of the subject being treated. These factors may be appropriately selected based on known techniques in the field.

The pharmaceutical composition in the present invention may further comprise pharmaceutically acceptable carriers as needed. "Pharmaceutically acceptable carrier" refers to additives commonly used in the field of pharmaceutical formulation. It includes excipients, binders, disintegrants, fillers, emulsifiers, flow regulators, and lubricants.

The excipient(s) includes sugars such as monosaccharides, disaccharides, cyclodextrins, and polysaccharides (more specifically, but not limited to, glucose, sucrose, lactose, raffinose, mannitol, sorbitol, inositol, dextrin, maltodextrin, starch, and cellulose), metal salts (e.g., sodium chloride, sodium phosphate or calcium phosphate, calcium sulfate, magnesium sulfate, calcium carbonate), citric acid, tartaric acid, glycine, low, medium, and high molecular weight polyethylene glycol (PEG), poloxamer (Pluronic), kaolin, silica, or combinations thereof.

The binder(s) includes starch paste using corn, wheat, rice, or potato starch, simple syrup, glucose solution, gelatin, tragacanth, methylcellulose, hydroxypropylmethylcellulose, sodium carboxymethylcellulose, shellac and/or polyvinylpyrrolidone.

The disintegrant(s) includes the aforementioned starch, lactose, carboxymethyl starch, cross-linked polyvinylpyrrolidone, agar, laminarin powder, sodium bicarbonate, calcium carbonate, alginic acid or sodium alginate, polyoxyethylene sorbitan fatty acid esters, sodium lauryl sulfate, monoglyceride stearate, or their salts.

The filler(s) includes the aforementioned sugars and/or calcium phosphate (e.g., tricalcium phosphate or dicalcium phosphate).

The emulsifier(s) includes sorbitan fatty acid esters, glycerin fatty acid esters, sucrose fatty acid esters, and propylene glycol fatty acid esters.

The flow regulator(s) and lubricant(s) include silicates, talc, stearates, or polyethylene glycol.

The carrier(s) is primarily used to facilitate the formation of the dosage form and to maintain the form and pharmacological effect, and they may be used as needed. In addition to the above additives, if necessary, flavoring agents, solubilizing agents, suspending agents, diluents, surfactants, stabilizers, absorption enhancers, bulking agents, wetting agents, moisturizing agents, adsorbents, disintegration inhibitors, coating agents, colorants, preservatives, antioxidants, fragrances, flavoring agents, sweeteners, buffers, etc., may also be included.

The pharmaceutical composition in the present invention may also comprise other drugs, provided that they do not lose their pharmacological effect. For example, in the case of injectable preparations, a predetermined amount of antibiotics may be comprised.

The form of the pharmaceutical composition in the present invention is not particularly limited provided that it does not inactivate the active ingredient drugs or other additional active ingredients. For example, it may be in liquid, solid, or semi-solid form. Specific forms include oral dosage forms such as liquids, powders, granules, tablets, capsules, sublingual tablets, troches, and non-oral dosage forms such as injectables, suspensions, emulsions, eye drops, nasal drops, creams, ointments, plasters, patches, and suppositories. When the composition comprises tyrosine kinase inhibitors, the preferred form is an oral dosage form.

The pharmaceutical composition in the present invention may be administered by any suitable route that does not deactivate the active ingredients. For example, it may be administered orally or non-orally (e.g., injection, aerosol, topical application, eye drops, nasal drops). Preferably, it is administered orally.

The pharmaceutical composition in the present invention preferably comprises the active ingredient(s) drug in amount(s) effective for the treatment and/or prevention of aneurysms without causing severe adverse event. For example, the total daily dose of the active ingredient drug(s) is preferably 0.0001 to 50 mg/kg body weight. The total dose of the active ingredient in existing pharmaceutical compositions of tyrosine kinase inhibitors related to PDGFRβ is approximately 0.1 to 5 mg/kg body weight per day for oral administration, and the pharmaceutical composition in the present invention may follow this dosage.

### <Method for Assisting Diagnosis>

The method for assisting diagnosis of an aneurysm in the present invention comprises the following steps:
- detecting a mutation in at least one gene of the PDGFRβ and AHNAK genes in a sample collected from a subject with an aneurysm; and
- predicting the treatment and/or preventive effect of a drug related to PDGFRβ or AHNAK on the subject.

Specifically, the method for assisting the diagnosis of an aneurysm further comprises:
- predicting that a PDGFRβ-related drug (especially a tyrosine kinase inhibitor) is effective in treating the aneurysm when a specific mutation in the PDGFRβ gene is confirmed to be present at a detectable level in the sample; or
- predicting that an AHNAK-related drug is effective in treating the aneurysm when a specific mutation in the AHNAK gene is confirmed to be present at a detectable level in the sample.

Here, "detectable level" may be defined as a level significantly higher than a predetermined threshold or a negative control, while the definition is not limited to those.

The method for assisting diagnosis of an aneurysm in the present invention is not particularly limited and may include methods for measuring circulating free DNA (cfDNA) in the blood, such as circulating tumor DNA (ctDNA), exosomes, microRNAs, proteins, etc., which are particles circulating in the blood derived from mutation-positive cells.

It has been clarified that somatic mutations in the PDGFRβ gene are associated with the onset of aneurysms, and that tyrosine kinase inhibitors are effective in treating and/or preventing aneurysms in patients with such mutations. Specifically, if somatic mutations in PDGFRβ are present at a detectable level in vascular tissue samples collected from subjects with aneurysms, it may be predicted that the effect of tyrosine kinase inhibitors may be particularly high.

It has been clarified that somatic mutations in the AHNAK gene are also associated with aneurysms. In patients with such mutations, AHNAK-related drugs, particularly FGF-R inhibitors, PKC inhibitors, and/or PI3K inhibitors, are predicted to be highly effective.

As described above, the method for assisting diagnosis in the present invention allows for predicting the treatment effect in patients before drug therapy, specifically non-surgical treatment, for aneurysms.

The "subject" in the method for assisting diagnosis in the present invention may be a human, a primate including a chimpanzee, a pet animal such as a dog or cat, a livestock animal such as cattle, horses, sheep, or goats, a rodent such as a mouse or rat, or an animal kept in a zoo. Preferably, the subject is a human, most preferably a human with an aneurysm or post-aneurysm treatment. Specific examples include patients with aneurysms before non-surgical treatment or those under observation after surgical treatment.

The "sample" in the method for assisting diagnosis in the present invention may be any sample collected from a subject with an aneurysm, such as body fluids (including blood (including plasma and serum), lymph fluid, urine, saliva, sweat, tissue fluid, body cavity fluid, cerebrospinal fluid, etc.) and vascular tissues (e.g., cerebral blood vessel tissues).

For detecting the target somatic mutation with high sensitivity, vascular tissue from the aneurysm-affected part (collected from the subject) may be used. When vascular tissue is used as the sample, the method for assisting diagnosis in the present invention is mainly useful for predicting the effect of preventing recurrence or treating recurrence.

Preferably, the body fluid sample should not comprise normal cells, and plasma, serum, or cerebrospinal fluid may be suitably used. Since it is known that nucleic acids derived from abnormal cells with somatic mutations are comprised in plasma or serum, the treatment and/or preventive effect of drugs related to PDGFRβ or AHNAK may be predicted by detecting the target somatic mutation from plasma or serum. When using body fluid samples, the method for assisting diagnosis in the present invention is not limited to subjects who have undergone surgical procedures and may be applied to asymptomatic subjects under observation, predicting the effect of preventing recurrence, treatment upon recurrence, and predicting the effect of non-surgical treatment.

In the method for assisting diagnosis in the present invention, when detecting mutations in the PDGFRβ gene, it is preferable to detect at least one mutation selected from the group consisting of p.559_562del, p.Y562N, p.Y562S, p.Y562D, and p.563_564del, as represented by the amino acid sequence of SEQ ID NO: 2. When detecting mutations in the AHNAK gene, it is preferable to detect at least one mutation selected from the group consisting of p.D1083Sfs20, as represented by the amino acid sequence of SEQ ID NO: 16.

The detection of mutated genes is not particularly limited, but may include analyzing the mRNA of the PDGFRβ gene or AHNAK gene comprised in the sample. In this case, the method may be carried out, wherein the cDNA of the PDGFRβ gene or AHNAK gene is prepared from total RNA in the sample and analyzed using existing methods such as RFLP, SSCP, TaqMan PCR, single-base extension, Invader, mass spectrometry, DNA array, pyrosequencing, and next-generation sequencers. Additionally, or alternatively, low-frequency mutations may be detected using DS.

The method for assisting diagnosis in the present invention comprises a step of predicting the treatment and/or preventive effect of tyrosine kinase inhibitors based on the detected gene mutations. For example, qualitatively, if the target gene mutation is detectable, it predicts a high effect of tyrosine kinase inhibitors. Quantitatively, it predicts a high effect if the ratio of the target gene mutation to normal genes exceeds a certain level. The prediction determination may be set based on predetermined criteria or using artificial intelligence (AI) constructed through machine learning with past prediction data and actual treatment performance data as training data.

### <Method for Treatment and/or Prevention>

The method for treating and/or preventing an aneurysm in the present invention comprises the administration of at least one of the following drugs to the subject:
i) drugs targeting platelet-derived growth factor receptor β (PDGFRβ);
ii) drugs than inhibit the function of desmoyokin (AHNAK); and
iii) drugs that inhibit signaling enhanced by mutations in PDGFRβ or AHNAK.

The subject for the method for treatment and/or prevention in the present invention may be a human, a primate including a chimpanzee, a pet animal such as a dog or cat, a livestock animal such as cattle, horses, sheep, or goats, a rodent such as a mouse or rat, or an animal kept in a zoo. Preferably, the subject is a human, most preferably a human with an aneurysm (also referred to as an "aneurysm patient").

When the subject is a human, it is preferable to target a human with any of the following gene mutations: : p.559_562del, p.Y562N, p.Y562S, p.Y562D, and p.563_564del as represented by the amino acid sequence of SEQ ID NO: 2; : p.D1083Sfs6, p.A2114T, p.G3819A, p.N3827S, p.E3850K, p.A4046V, p.D4701E, and p.H5817Rfs20 as represented by the amino acid sequence of SEQ ID NO: 16.

The method for treatment and/or prevention in the present invention comprises the administration of drugs related to PDGFRβ and/or AHNAK to the subject. A PDGFRβ-related drug is preferably a tyrosine kinase inhibitor. The dosage of the drug in the method for treatment and/or prevention in the present invention varies depending on various conditions such as the effect and stability of the drug, dosage form, type of carrier used, administration method, and condition of the subject. These may be appropriately selected based on known techniques in the field.

The method for treatment and/or prevention in the present invention may also comprise the combination of another drug in addition to the above drug. For example, an antibiotic may be used in combination.

The dosage form of the drug administered in the method for treatment and/or prevention in the present invention is not particularly limited provided that it does not inactivate the drug itself and other additional active ingredients. For example, it may be in liquid, solid, or semi-solid form. Specific dosage forms include, for example, oral dosage forms such as liquid, powder, granules, tablets, capsules, sublingual, and lozenges, or parenteral dosage forms such as injectable, suspension, emulsion, ophthalmic, nasal spray, cream, ointment, hard plaster, sachet, and suppository. In the case of administration of tyrosine kinase inhibitors, it is preferable to use oral dosage forms.

The administration route of the drug in the method for treatment and/or prevention in the present invention is not particularly limited provided that the administered active ingredient is not inactivated. For example, oral administration, parenteral administration (e.g., intravenous injection, subcutaneous injection, and intramuscular injection), and topical administration (e.g., percutaneous administration and intranasal administration) may be used. These administration methods may be appropriately selected depending on the type of drug and the condition of the subject.

The methods of treatment and/or prevention in the present invention preferably administer the active ingredient, i.e., the drug, in an amount that is effective in treating and/or preventing aneurysms and that does not cause severe adverse events. For example, the total dosage of the active ingredient(s), or drug(s), may be 0.0001 to 50 mg/kg body weight/day. Furthermore, more preferable dosages within the above range may be made: existing commercially available pharmaceuticals for tyrosine kinase inhibitors targeting PDGFRβ are mainly used for the treatment of malignant tumors, and the dosage of the active ingredient in humans is about 0.1 to 3 mg/kg body weight/day for oral administration. FGF-R inhibitors, PKC inhibitors, and PI3K inhibitors are also commercially available and developed mainly for the treatment of malignant tumors, and the dosages of the active ingredients in pharmaceuticals commercially available or in development are in the range of about 0.1-5 mg/kg body weight/day. In the methods for treatment and/or prevention in the present invention, the total dosage of the drugs may be the same as that of the pharmaceuticals commercially available or in development, or it may be a smaller dosage. For example, the total dosage may be 0.0001 to 2.5 mg/kg body weight/day, 0.001 to 2.5 mg/kg body weight/day, 0.01 to 2.5mg/kg body weight/day, 0.1 to 2.5 mg/kg body weight/day, 0.0001 to 0.5 mg/kg body weight/day,0.001 to 0.5mg/kg body weight/day, 0.01 to 0.5mg/kg body weight/day, 0.0001 to 0.05 mg/kg body weight/day, or 0.001 to 0.05 mg/kg body weight/day. In the treatment and/or prevention of aneurysms, it is assumed that the dosage of the above active ingredients will provide at least sufficient treatment/preventive effect than the dosage for the treatment of malignant tumors. It is a very important advantage that a higher effect may be obtained with a smaller dosage in terms of reduction of adverse events of the drug, reduction of treatment/prevention costs, etc.

### <Methods for Determining the Treatment Effectiveness of Drugs in Treatment of Aneurysms >

A method for determining treatment effect of a drug on an aneurysm in the present invention (hereinafter referred to simply as "method for determination") is characterized in that it comprises the following steps b) to d):
(b) contacting human cells with the drug *in vitro*;
(c) measuring the amount(s) of phosphorylated extracellular signal-regulated kinase (p-ERK) and/or the amount of phosphorylated tyrosine for the cells of step b); and
(d) predicting the treatment effect of the drug on an aneurysm from the measurement value(s) in step c).

Alternatively, another aspect of the method of determination in the present invention is characterized in that it comprises the following steps a') to d'):
a') preparing human cells into which a mutated PDGFRβ gene has been introduced;
b') contacting the cells with the drug *in vitro*; and
c') measuring the amount(s) of phosphorylated extracellular signal-regulated kinase (p-ERK) and/or phosphorylated tyrosine for the cells of step b'); and
d') predicting the treatment effect of the drug on an aneurysm from the measurement value(s) from process c').

According to the present invention, it is possible to determine *in vitro* the treatment effect of a drug whose treatment effect on aneurysms is unknown. In particular, this method may be useful for screening therapeutic drugs for aneurysms.

### Step a') Preparation of Cells with Mutated PDGFRβ Gene

The method for determination in the present invention may comprise a step a') for preparing human cells introduced a mutated PDGFRβ gene. The "human cells introduced a mutated PDGFRβ gene" (hereinafter referred to as "PDGFRβ mutant cells") used in this step may be prepared by introducing a mutated PDGFRβ gene (hereinafter referred to as "mutated PDGFRβ gene") by any known method. The human cells used are not particularly limited, and any known human cell lines may be used. In particular, immortalized cells are preferred, and examples include HEK293T cells, NIH3T3 cells, HUVEC cells, and the like.

The mutations in the PDGFRβ gene are preferably those corresponding to at least one mutation selected from the group consisting of p.559_562del, p.563_564del, p.Y562N, p.Y562S, and p.Y562D in the amino acid sequence represented by SEQ ID NO: 2. In other words, in the nucleotide sequence represented by SEQ ID NO: 1, the PDGFRβ gene preferably has at least one mutation selected from c.1684T>A (p.Y562N), c.1684T>G (p.Y562D), c.1685A>C (p.Y562S), c.1675_1686del (p.559_562del), and c.1687_1692del (p.563_564del). Such mutated genes (nucleic acids, preferably DNA) may be prepared by known methods such as chemical synthesis, PCR or the like. The "PDGFRβ gene" herein does not need to have the full length of the nucleotide sequence represented by SEQ ID NO: 1, provided that it comprises the target mutation region and encodes a protein that retains tyrosine kinase activity. It may also be a nucleic acid fragment having a part of the nucleotide sequence represented by SEQ ID NO: 1.

The DNA encoding the target mutation may be introduced into human cells using known gene expression vectors. The gene expression vector comprises at least a promoter and the mutated PDGFRβ gene downstream, and may express the PDGFRβ mutant in human cells. As used herein, the term "expressible state" refers to the state in that the gene to be expressed is placed downstream of the promoter under the control of the promoter. The gene expression vector used in this step is a vector comprising the mutated PDGFRβ gene in an expressible state and may express the mutated PDGFRβ gene in somatic cells.

Vectors that may be used as gene expression vectors are not particularly limited, provided that they may express the PDGFRβ mutant in somatic cells. Examples include viral vectors, plasmid vectors, and artificial chromosome vectors. Examples The viral vectors that may be used as gene expression vectors include adenovirus vectors, adeno-associated virus (AAV) vectors, retrovirus vectors, lentivirus vectors, and Sendai virus vectors. The plasmid vectors that may be used as gene expression vectors include plasmids derived from E. coli (pBR322, pUC18, pUC19, pUC118, pUC119, pBluescript, etc.), plasmids derived from actinomycetes (pU486, etc.), plasmids derived from Bacillus subtilis (pUB110, pSH19, etc.), plasmids derived from yeast (YEp13, YEp24, YCp50, etc.), and commercially available vectors. Examples of artificial chromosome vectors that may be used as gene expression vectors include human artificial chromosomes (HAC), yeast artificial chromosomes (YAC), and bacterial artificial chromosomes (BAC, PAC).

Promoters comprised in the gene expression vector may be, for example, CMV promoter (CMV-IE promoter), SV40 early promoter, RSV promoter, HSV-TK promoter, EF1α promoter, Ub promoter, metallothionein promoter, SRα promoter, or CAG promoter. Other examples include inducible promoters such as heat shock promoters that may be controlled by temperature and tetracycline-responsive promoters that may be controlled by the presence of tetracycline.

The gene expression vector may also comprise selectable marker genes, reporter genes, control sequences (expression control sequences, intron sequences, nuclease recognition sequences, replication origin sequences), etc. The expression control sequences include enhancers, ribosome binding sites, terminators, and polyA addition signals. Examples of nuclease recognition sequences include restriction enzyme recognition sequences, loxP sequences recognized by Cre recombinase, target sequences for artificial nucleases such as ZFN and TALEN, and target sequences for the CRISPR/Cas9 system. Examples of replication origin sequences include SV40 replication origin sequences.

Selectable marker genes comprised in the gene expression vector are selectable marker genes that may select somatic cells into which the gene expression vector has been introduced. Specific examples of selectable marker genes include antibiotic resistance genes such as ampicillin resistance gene, kanamycin resistance gene, tetracycline resistance gene, chloramphenicol resistance gene, neomycin resistance gene, puromycin resistance gene, or hygromycin resistance gene.

Reporter genes comprised in the gene expression vector are genes that encode for reporters that may identify somatic cells into which the gene expression vector has been introduced. Examples of reporter genes include genes coding for fluorescent proteins such as GFP and RFP, and luciferase genes.

Methods for introducing gene expression vectors into human cells are not particularly limited and may be appropriately selected depending on the type of gene expression vector (plasmid vector, viral vector, etc.). For example, the mutated PDGFRβ gene may be introduced into cells by methods such as viral infection, lipofection, liposome method, electroporation, calcium phosphate method, and DEAE-Dextran method.

PDGFRβ mutant cells may be cultured in known media suitable for animal cell culture. Examples of such media comprise DMEM (Dulbecco's Modified Eagle Medium), BME medium, BGJb medium, CMRL1066 medium, Glasgow MEM medium, Improved MEM Zinc Option medium, IMDM (Iscove's Modified Dulbecco's Medium), Medium 199, Eagle MEM medium, αMEM medium, Ham F10 medium, Ham F12 medium, RPMI 1640 medium, Fischer's medium, and mixtures of these media, but are not limited thereto.

Culture vessels such as plates, dishes, and flasks may be used for culturing. The culture conditions are not particularly limited, but typically, culturing is conducted at 37°C under a 5% CO₂ atmosphere. The culture period is not particularly limited, but may be about 10 to 72 hours, particularly about 12 to 24 hours.

### Step b) or Step b') Drug Contact Step

The method for determining in the present invention comprises a step of contacting the cells with a drug *in vitro.* In this step, it is necessary to add the target drug to the human cell culture medium or the culture medium from step a'). A medium exchange may be performed at the time of addition of drug or immediately before. The target drug is not particularly limited provided that it is a drug whose treatment effect on aneurysms needs to be determined. The target drug may be, for example, a drug known to be related to PDGFRβ, such as a known tyrosine kinase inhibitor. The addition of the drug to the medium may be performed by preparing a stock solution of the target drug at a high concentration in a solvent such as water or DMSO and then adding it to the medium. The concentration of the drug added to the medium may be appropriately determined based on the solubility, effective therapeutic concentration, toxicity, etc., of the drug used, and may be, for example, about 1 nM to 1000 nM or 10 nM to 100 nM. In this step, it is preferable to use multiple types of media containing various concentrations of the drug or two types of media, one containing the drug and one not containing the drug, for cell culture.

The contact between the cells and the drug is performed by adding the drug to the medium containing the cells and then incubating it at 37°C under 5% CO₂ conditions. The incubation time is not particularly limited and may be, for example, about 5 minutes to 4 hours, particularly about 1 to 3 hours.

### Step c) and Step c') Measurement of Phosphorylated ERK and/or Phosphorylated Tyrosine

The method for determining in the present invention comprises a step of measuring the amount of phosphorylated ERK (p-ERK) and/or phosphorylated tyrosine (p-Tyr) in the cells from step b) or step b'). This step may have the following procedures. After removing the medium from the culture, the remaining cells are lysed on ice using a lysis solution (e.g., RIPA buffer) to obtain a protein solution. The p-ERK and/or p-Tyr in the obtained solution may be quantified using known protein measurement methods such as Western blot, immunoassay (e.g., ELISA), or mass spectrometry. Western blot and immunoassays may be performed using anti-p-ERK and/or anti-p-Tyr antibodies.

### Step d) or Step d') Prediction of Aneurysm Treatment Effect

The present invention comprises a step of predicting the treatment effect of the drug on aneurysms based on the measurement values of p-ERK and/or p-Tyr obtained in step c) or step c'). ERK is a protein related to the ERK pathway, one of the mitogen-activated protein kinase (MAPK) signaling pathways, and is known to be involved in cell proliferation and differentiation, etc. In the ERK pathway, the phosphorylation of ERK is involved in signal transduction, and it is known that excessive phosphorylation may induce abnormal cell proliferation. The present inventor has confirmed that ERK phosphorylation is enhanced in cells introduced with the mutated PDGFRβ gene. Furthermore, it was confirmed that in the presence of multiple drugs, the amount of p-ERK was significantly decreased to the same level as in cells introduced with the wild-type PDGFRβ gene. Therefore, this step comprises confirming whether the measured p-ERK amount falls below a preset value, decreases dose-dependently with the drug, or is significantly lower than in cells cultured without the drug. If any of these conditions are met, it is predicted that the target drug has a treatment effect on aneurysms.

The present inventor has confirmed that tyrosine phosphorylation is enhanced in cells introduced with the mutated PDGFRβ gene, indicating that the mutation significantly enhances tyrosine kinase activity itself. This step comprises measuring the amount of p-Tyr along with or instead of the amount of p-ERK. In this case, this step comprises confirming whether the measured amount of p-Tyr falls below a preset value, decreases dose-dependently with the drug, or is significantly lower than in cells cultured without the drug. If any of these conditions are met, it is predicted that the target drug has a treatment effect on aneurysms.

### [Examples]

The present invention is explained in detail with the examples below, but it is not intended to limit the invention to the scope of the examples.

### <Reference Example 1: Analysis of Somatic Mutations in Basilar Artery Tissue of Cerebral Aneurysm>

### 1. Sample Collection from Cerebral Aneurysm Patients

Blood samples were collected from 69 patients diagnosed with cerebral aneurysm (CA) having either saccular aneurysm (SCA) or fusiform aneurysm (FCA) and who underwent surgical procedures for clipping and/or reconstruction of the aneurysm artery. Basilar artery tissue samples were obtained from tissue pieces resected during surgery. The patients had no family history of CA and no history of hereditary vascular diseases. The collected tissue and blood samples were rapidly frozen in liquid nitrogen and stored at -80°C.

### 2. Whole Exome Sequencing

DNA was purified from the basilar artery tissue sample and blood sample of each patient. After fragmentation with Covaris, a library was prepared using 30-50 ng of normal lymphocyte DNA with the KAPA HyperPrep Kit (Kapa Biosystems). Exome capture was performed using the SureSelect Human All Exon V6 (Agilent Technologies). Sequencing was conducted using the Illumina HiSeq2500 SBS V4 with 125 bp paired-end reads. The average depth of sequencing was 126x for CA and 100x for normal lymphocyte DNA.

### 3. Targeted Deep Sequencing for Target Sequence Determination

To verify and detect low variant allele frequency (VAF) mutations in CA, RNA capture probes (SureSelect) targeting the entire coding regions of 65 genes identified by whole exome sequencing or related to CA or cerebrovascular diseases were created. Sequence libraries were constructed from 50 ng of cDNA using SureSelect XT low input reagents with molecular barcodes. Target capture was performed using the SureSelect XT Target Enrichment System. Sequencing of the target capture library was performed with HiSeq2500 using 125 bp paired-end reads. The average depth of the target regions after removing duplicates using molecular barcodes was 1500x.

### 4. Mutation Calling Using WES and DS Data

Somatic mutations were extracted from WES data using the Genomon pipeline (https://Genomon-project.github.io/GenomonPages/). Paired-end reads were mapped to the human reference genome GRCh37 using BWA-MEM, and PCR amplicons were marked using biobambam. Somatic SNVs and indels were called by comparing paired CA and blood samples. Data were filtered using Fisher's exact test with the following criteria: read depth in both CA and blood samples ≥8; base quality score ≥15; mapping quality score ≥20; variant-supporting reads in CA samples ≥4; VAF in CA samples ≥2%; VAF in blood samples ≤10%; and Fisher's exact test p-value ≤0.1. Mutations were further filtered using EBCall (ref) normal tissue panel sequence data and annotated with ANNOVAR.

For targeted sequencing, the resulting fastq files were analyzed on an Agilent SureCall 3.5 (Agilent) using the edit settings (allele frequency: >0.5%, number of read pairs per barcode: >1). Clean fastq files were mapped to the human reference genome version GRCh37 (hg19) using BWA-MEM. SNVs were called using SNPPET SNP caller. 250 or more total reads and 5 or more SNV reads. with a total of >250 reads and >5 SNV reads, were used in the analysis. Raw-data for variants were annotated with SureCall default settings and filtered by removing common variants in the pooled targeted sequencing data of 12 blood samples and 2 vessel controls. In addition, germline variants detected by whole exome sequencing of blood DNA in the same patients were removed from these variants. These data were visualized and analyzed using R package Maftools.

### 5. Germline Mutation Calling

Germline SNVs and indels were called using Genome Analysis Toolkit version 3.8 (http://software.broadinstitute.org/gatk/). Normal tissue BAM files from exome and WGS which had been used for the somatic mutation call were used for base quality score recalibration and HaplotypeCaller. Called variants were filtered by performing variant quality score recalibration, retaining splicing or exonic variants, and removing common variants (allele frequency >1%).

### 6. Pathway Analysis of Mutant Genes in CA

To investigate the relationship between overlapping genes and cerebral aneurysms, network analysis was conducted using Ingenuity Pathway Analysis (IPA) (Ingenuity Systems, Qiagen, Redwood City, CA). A list of 13 genes identified by WES was uploaded to IPA, and network analysis was performed using the default filtering and selected species (human, mouse, rat) with the keyword "Aneurysm."

### 7. Results

WES was performed on CA and blood samples from all 69 cases, with an average depth of sequencing of 117x and 92x, respectively. For 45 filtered cases, 1761 nonsynonymous SNVs and 38 indels in 405 genes were detected. With the mutation call, Significant mutated genes identified by WES comprised 381 genes with somatic mutations in saccular aneurysms (SCA) and 29 genes with somatic mutations in fusiform aneurysms (FCA). Figure 2 shows a Venn diagram of characteristic common somatic mutations. It was found that the genes for platelet-derived growth factor receptor β (PDGFRβ and AHNAK nucleoprotein (AHNAK) had somatic mutations in both FCA and SCA, with PDGFRβ having the most mutations.

Targeted deep sequencing (DS) was performed on tissue samples obtained from 68 CA tissues along with two normal cortical arteries and 10 blood samples to verify WES results and detect low-frequency target alleles in the samples. After removing germline mutations and filtering, 473 somatic mutations in 40 genes were detected in 62 patients. Among these, 16 genes were identified as having highly reproducible somatic mutations observed in multiple cases. Pathway analysis identified PDGFRβ and AHNAK from these 16 genes (Figures 3 and 4). Figures 1 and 11 show the positions and contents of PDGFRβ and AHNAK mutations detected by WES. Four types of PDGFRβ mutations (p.Y562N, p.Y562S, p.559_562del, and p.563_564del) were detected in four cases. Three types of AHNAK mutations (p.G3819A, p.N3827S, and p.D4701E) were detected in four cases.

DS of PDGFRβ in the validation test of 68 cases of aneurysm and arterial walls revealed five mutations in five cases: three missense mutations (p.Y562N, p.Y562S, p.Y562D), a four-amino acid in-frame deletion in exon 12 (p.559_562del), and a two-amino acid in-frame deletion (p.563_564del). In summary, four mutations (p.Y562N, p.Y562S, p.559_562del, and p.563_564del) were detected in four cases by both WES and DS, and one mutation (p.Y562D) was detected in one case by DS only.

Similarly, DS was performed for AHNAK. Five mutations were identified in eight cases: three missense mutations (p.E3850K, p.A2114T, and p.A4046V) and two frameshift mutations (p.D1086Sfs6 and p.H5817Rfs20).

As described above, WES and DS of DNA from cerebral aneurysms and normal tissues demonstrated that PDGFRβ mutations had the highest incidence, suggesting that this gene plays a synergistic causal role in the formation of fusiform and saccular aneurysms in the brain.

### <Reference Example 2: Immunohistochemical Study of Cerebral Aneurysm Tissue>

### 1. Preparation of Frozen Sections

Cerebral aneurysm tissue (basilar artery wall) samples were embedded in Tissue-Tek O.C.T. compound (Sakura Finetek) and frozen in liquid nitrogen-cooled isopentane. Tissue sections were cut continuously at a thickness of 10 µm using a cryostat (CM3050S, Leica) set at -20°C. Sections were air-dried overnight at room temperature and stored at -80°C.

### 2. Localization of Gene Mutations in Cerebral Aneurysm and Vascular Structure

Microdissection of vascular layers (intima, media, and adventitia) was performed using a laser-assisted microdissection system (LMD7000, Leica) or manually under a microscope for five cases (FU2, FU3, FU6, FR1, SU50). The sizes of the cerebral aneurysms in each case are shown in Table 1. For laser-assisted microdissection, 50 µm frozen sections were placed on PEN membrane slides (2.0 µm, Leica) pre-coated with 0.1% poly-L-lysine solution (Fujifilm Wako Pure Chemical, Japan) and fixed with ethanol before staining with 0.05% toluidine blue. Desired regions were cut with a laser beam under conditions of 6.3x magnification, 28 power, 16 aperture, 9 speed, sample balance 3, head current 100%, and 800 pulse frequency. For manual dissection, 10 µm frozen sections were stained with toluidine blue and excised manually with a blade under a stereomicroscope (10x, MZ-95, Leica). Processed sections were collected in tubes by vascular layer. Dissected sections were suspended in 180 µL of ATL buffer (Qiagen) comprising 0.2 mg/mL proteinase K (Qiagen). After incubation at 56°C for 18 hours, genomic DNA was extracted using the QIAamp DNA Micro Kit (Qiagen). The concentration of double-stranded DNA was measured using a Qubit fluorometer (Qubit 2.0, Thermo Scientific). Whole exome sequencing was performed on the extracted DNA using the same method as described in Reference Example 1.

**[Table 1]**

| (medical) Case | Classification. | Aneurysm diameter (mm) |
|---|---|---|
| FU2 | Small Spindle-shaped | 6.95 |
| FU3 | Small Spindle-shaped | 4.74 |
| FU6 | Medium scale Spindle-shaped | 7.3 |
| FR1 | Medium scale Spindle-shaped | 11 |
| SU50 | Large scale Cystic | 22 |

### 3. Results

The inventor examined which vascular layer expressed six PDGFRβ mutant DNAs from the DNA obtained from each of the three layers (intima, media, and adventitia) of each sample. The results showed that mutations were most frequently observed in the adventitial layer in small fusiform aneurysms, in the medial layer in medium and large fusiform aneurysms (Figure 5). In samples of giant aneurysms, PDGFRβ mutations were observed in both the medial and intimal layers. These findings suggest that the formation of cerebral aneurysms caused by gene mutations is due to the introduction of a mutation in one of the cells in the adventitial layer of normal blood vessels. It was considered that the initial small fusiform aneurysm was formed within the adventitial layer as the pathological process progressed.

### <Example 1: Testing Human Cells with Introduced PDGFRβ Mutations>

### 1. Plasmid Preparation

Human *PDGFRβ* was amplified using PCR from cDNA derived from SH-SY-5Y cells and cloned into PCR-Blunt II TOPO (Thermo Fisher Scientific). To prepare *PDGFRβ* mutants, six mutations (c.1684T>A (p.Y562N), c.1684T>G (p.Y562D), c.1685A>C (p.Y562S), c.1685A>G (p.Y562C), c.1675_1686del (p.559_562del), and c.1687_1692del (p.563_564del)) were separately introduced into *PDGFRβ*-TOPO using PCR-based mutagenesis with PrimeSTAR MAX DNA Polymerase (Takara). Fragments of wild-type *PDGFRβ* or *PDGFRβ* mutants were obtained by PCR from *PDGFRβ* wild-type-TOPO or *PDGFRβ* mutant-TOPO, respectively, to prepare *PDGFRβ* wild-type-*IRES2*-*eGFP* or *PDGFRβ* mutant*-IRES2-eGFP.* The *IRES2-eGFP* fragment was obtained by PCR from pCAG-Cre-*IRES2*-*GFP* plasmid (Addgene, #26646). These fragments were assembled into the pCAGGS vector (RDB08938, RIKEN BioResource Center) using NEBuilder HiFi DNA Assembly (New England BioLabs). The primers used for mutagenesis and assembly are shown in Table 2. *PDGFRβ-HA* was amplified by PCR using pAAN-CMV-*PDGFRβ*-*HA* plasmid as a template. *PDGFRβ* (BspI-NotI)-HA was subcloned into PCR-Blunt II TOPO. The obtained plasmid was digested with BspEI and EcoRV to remove the HA-coding fragment. This fragment was cloned into *PDGFRβ* wild-type-*IRES2*-*eGFP* or *PDGFRβ* mutant*-IRES2-eGFP* digested with BspEI-EcoRV to prepare *PDGFRβ* wild-type*-HA-IRES2-eGFP* or *PDGFRβ* mutant-HA-IRES2-eGFP.

**[Table 2]**

| Variation | | SEQ ID NO: |
|---|---|---|
| c.1675_1686del; p.559_562del | | |
| Forward primer | 5'-GCAGAAGGAGATCCGATGGAAGGTG-3' | 3 |
| Reverse primer | 5'-CGGATCTCCTTCTGCCAAAGCATGAT-3' | 4 |

| c.1687_1692del; p.563_564del | | |
|---|---|---|
| Forward primer | 5'-ACGTTACCGATGGAAGGTGATTGAG-3' | 5 |
| Reverse primer | 5'-TTCCATCGGGTAACGTGGCTTCTTCTG-3' | 6 |

| c.1684T>A; p.Y562N | | |
|---|---|---|
| Forward primer | 5'-GCCACGTAACGAGATCCGATGGAAGG-3' | 7 |
| Reverse primer | 5'-ATCTCGTTACGTGGCTTCTTCTTCTGCCA-3' | 8 |

| c.1685A>C; p.Y562S | | |
|---|---|---|
| Forward primer | 5'-CCACGTTCCGAGATCCGATGGAAGGT-3' | 9 |
| Reverse primer | 5'-GATCTCGGAACGTGGCTTCTTCTGCC-3' | 10 |

| c.1685A>G; p.Y562C | | |
|---|---|---|
| Forward primer | 5'-CCACGTTGCGAGATCCGATGGAAGGT-3' | 11 |
| Reverse primer | 5'-GATCTCGCAACGTGGCTTCTTCTGCC-3' | 12 |

| c.1685A>G; p.Y562D | | |
|---|---|---|
| Forward primer | 5'-GCCACGTGACGAGATCCGATGGAAGG-3' | 13 |
| Reverse primer | 5'-ATCTCGTCACGTGGCTTCTTCTTCTGCCA-3' | 14 |

### 2. Cell Culture and Gene Mutation Introduction

Human embryonic kidney (HEK) 293T cells were obtained from RIKEN BioResource Research Center (Japan) and cultured in Dulbecco's Modified Eagle Medium (DMEM, low glucose, Gibco) comprising 10% fetal bovine serum (FBS, Nissui BioScience, Japan), 100 U/mL penicillin-streptomycin (Gibco), and non-essential amino acid solution (Gibco) at 37°C in a humidified incubator with 5% CO2. Cells were seeded in 24-well plates (2×10⁵ cells/well) and incubated at 37°C with 5% CO2 for 16 hours. Plasmid DNA (1.0 µg DNA/well) was transfected using Lipofectamine 3000 (Thermo). After 5 hours, the medium was replaced with fresh medium and incubated at 37°C with 5% CO2 for 16 hours.

### 3. Western Blot Analysis

After culture, the culture medium was removed from the wells, and ice-cold RIPA buffer (25 mM Tris-HCl, pH 7.6, 150 mM NaCl, 1% NP-40, 1% sodium deoxycholate, 0.1% SDS) comprising cOmpleteTM, ULTRA protease inhibitor (Roche), PhosSTOPTM phosphatase inhibitor (Roche), and 0.016 U/µL Benzonase (Novagen) was added to the wells. Cells were then incubated on ice for 15 minutes. The lysates were centrifuged at 15,000 × g for 10 minutes at 4°C to obtain clear supernatants. Protein concentration was measured using the BCA Protein Assay Kit (Takara Bio). Ten micrograms of cell lysates were separated by electrophoresis on 10% or 5-20% SDS-PAGE gels (Real Gel Plate, BIOCRAFT) and transferred to a polyvinylidene difluoride (PVDF) membrane (Immobilon-P, 0.45 µm, Merck). The membrane was blocked with may get signal (Toyobo) or 5% bovine serum albumin in TBS-0.1% Tween 20 (TBST) for 1 hour at room temperature. The membrane was then incubated with primary antibodies in may get signal solution 1 (Toyobo) at 4°C for 14-18 hours. The primary antibodies used were:
Rabbit anti-PDGFRB monoclonal antibody (Y-92, Abcam)
Mouse anti-phosphotyrosine monoclonal antibody (P-Tyr-100, Cell Signaling Technology)
Rabbit anti-phospho-ERK1/2 monoclonal antibody (D13.14, Cell Signaling Technology)
Rabbit anti-GFP polyclonal antibody (MBL)
Mouse anti-ERK1/2 monoclonal antibody (L34F12, Cell Signaling Technology)
Rabbit anti-phospho-AKT monoclonal antibody (D9E, Cell Signaling Technology)
Rabbit anti-AKT monoclonal antibody (C67E7, Cell Signaling Technology)
Rabbit anti-phospho-STAT3 polyclonal antibody (Tyr705, Cell Signaling Technology)
Rabbit anti-STAT3 monoclonal antibody (D3Z2, Cell Signaling Technology)
Mouse anti-β-actin monoclonal antibody (8H10D10, Cell Signaling Technology)

Next, the membrane was incubated with goat anti-mouse HRP-conjugated antibody (Jackson ImmunoResearch) or donkey anti-rabbit HRP-conjugated antibody (Jackson ImmunoResearch) in Can Get Signal (R) solution 2 at room temperature for 1 hour. Proteins on the membrane were detected using Chemi-Lumi One L (Nacalai Tesque).

### 4. Evaluation of Tyrosine Kinase Inhibitors on 293T Cells

Transfected 293T cells were incubated in serum-free medium for 16 hours, followed by the addition of recombinant human PDGF-BB (Cell Signaling Technology) and incubation at 37°C with 5% CO₂ for 15 minutes. Recombinant human PDGF-BB was diluted serially in Opti-MEM medium (Gibco) after being dissolved at 50 g/mL in 20 mM, pH 3.04. The final concentration of PDGF-BB was 25-6.25 ng/mL.

Tyrosine kinase inhibitors (TKIs) sunitinib, axitinib, and dasatinib were obtained from Selleck Biotech (Japan). Each TKI was dissolved at 10 mM in DMSO and diluted serially in Opti-MEM medium before being added to each well. The final concentration of TKIs was 10 nM or 100 nM, with a final DMSO concentration of 0.01%. Plates with added TKIs were incubated at 37°C with 5% CO₂ for 3 hours. At the end of PDGF-BB or TKI treatment, the plates were removed from the incubator and cooled on ice. Western blotting was performed to detect PDGFRβ in each well.

### 5. Results

Mutant and wild-type constructs were transfected into HEK293T cells, and phosphorylation signals were examined. In cells with the wild-type construct, three proteins (about 180 kDa, about 160 kDa, and about 130 kDa) were detected by Western blotting using anti-PDGFRβ antibody (Figure 6). Total PDGFRβ levels were calculated by summing the intensities of the up to 180, up to 160, and up to 130 kDa bands and normalized to β-actin (AKT). Cells with mutant constructs had a small amount of the 180 kDa protein, but the 160 kDa and 130 kDa proteins were similar in amount to those in cells with the wild-type construct. In cells with mutant constructs, activation of phosphorylation in downstream signaling proteins was significantly increased in ERK.

In the case of wild-type constructs, PDGFRβ phosphorylation increased with the amount of added PDGF-BB, but mutant constructs did not respond (Figure 7). This suggests that PDGFRβ mutants are independent of ERK-activating ligands.

PDGFRβ phosphorylation was significantly inhibited by the tyrosine kinase inhibitors sunitinib (Figure 8A), axitinib (Figure 8B), and dasatinib (Figure 8C). Since ERK phosphorylation was hardly observed in normal cerebral arteries (data not shown), increased/excessive ERK phosphorylation in aneurysm tissue may serve as an appropriate marker for detecting mutations.

### <Example 2: Study in an Animal Model with Introduced PDGFRβ Mutations (I)>

### 1. Construction of Animal Model

A mouse CA (cerebral aneurysm) model was constructed by applying adeno-associated virus (AAV) around the non-branching region of the basilar artery. HEK293T cells were transfected with pHelper vector, pRC vector, and a pAAV vector comprising the target gene. After culturing, AAV was harvested using the iodixanol gradient method. After determining the AAV serotype, it was mixed with a hydrogel of PBS comprising 15% Pluronic F-127. The virus concentrations (vg/mL) in the hydrogels of AAV-CMV-EGFP, AAV-CMV-PDGFRβ, and AAV-CMV-PDGFRβ p559_562del were 2×10¹³, 1×10¹², and 1×10¹², respectively.

Male C57BL/6N mice aged 8-11 weeks (CLEA Japan) were deeply anesthetized with medetomidine (0.3 mg/kg), midazolam (4 mg/kg), and butorphanol (5 mg/kg) administered intraperitoneally (i.p.). The basilar artery was exposed as previously described (Yonekura et al., 2004). Three microliters of hydrogel were applied to the basilar artery and removed after 5 minutes. This application was repeated two more times. The mice were divided into three subgroups (control, PDGFRβ(wt), and PDGFRβ(mutant)). Sunitinib, axitinib, and dasatinib were dissolved in a vehicle (2% DMSO, 15% PEG300) and administered daily by i.p. injection at 40, 25, and 10 mg/kg/day, respectively. The mice were deeply anesthetized with isoflurane and decapitated. The shaved heads were immediately transferred to 0.1 M phosphate buffer comprising 4% paraformaldehyde and incubated at 4°C for at least 24 hours. The brainstem comprising the basilar artery was removed from the fixed skull, incubated with the fixative at 4°C for another 24 hours, and then embedded in paraffin. The brain area from 1 mm rostral to the cranial base joint to 100 mm caudal was cut into 4 mm sections.

### 2. Histopathology and Immunohistochemistry

To evaluate the effect of the virus, sections were stained with Elastica Van Gieson (EVG) method, selecting the largest artery section from each brain sample for measurement. The internal elastic membrane of the vascular cross-section was examined using EVG staining (dark purple staining) with chicken anti-GFP antibody (Aves GFP-1000), goat anti-alpha SMA antibody (abcam ab21027), and anti-PDGFRβ antibody (abcam ab32570). The area and perimeter of the stained sites were calculated using FUJI-ImageJ software (J. Schindelin et al., Nature Methods, 2012).

### 3. Results

To demonstrate that activation of the PDGFRβ signaling pathway may indeed induce aneurysms, an animal model of aneurysms was developed by introducing mutant genes into the basilar artery tissue of mice using adeno-associated virus (AAV). AAV coding for either wild-type or mutant (p.559-562del) PDGFRβ was mixed with AAV coding for eGFP and applied to the basilar artery of mice. One week post-infection, proteins derived from the AAV gene were expressed in the vascular layer surrounding the basilar artery. Additionally, viral infection was observed in adjacent tissues such as the ventral surface of the brainstem, arachnoid, and dura mater. At 28 days post-infection, the mice were sacrificed, and the size of the basilar artery was compared to animals infected with AAV expressing only eGFP. Basilar arteries infected with AAV for mutant PDGFRβ were significantly enlarged compared to those infected with eGFP virus alone. Conversely, no such enlargement was observed in arteries infected with AAV for wild-type PDGFRβ. These results suggested that the primary factor in the expansion of the basilar artery is not overexpression of PDGFRβ but its enzymatic activity.

To investigate the role of tyrosine kinase activity of mutant PDGFRβ in the expansion of the basilar artery, tyrosine kinase inhibitors were administered to mice infected with AAV for mutant PDGFRβ. Mice treated with sunitinib showed smaller basilar arteries compared to those treated with vehicle alone (Figure 9). The inhibition of enlargement by sunitinib was statistically significant (unpaired non-parametric Mann-Whitney U test, p<0.05). Similar results were observed with other tyrosine kinase inhibitors, axitinib, and dasatinib.

### <Example 3: Study in an Animal Model with Introduced PDGFRβ Mutations (II)>

Following the same method as in Example 2, an animal model of cerebral aneurysm with introduced PDGFRβ mutation (p.559_562del) was constructed. From day 7 to day 28 after mutation introduction, axitinib, dasatinib, or sunitinib solutions (98.81 µM in 2% DMSO, 15% PEG300) were administered daily by i.p. injection (20 µL each). The administration doses were 0.76 mg/kg/day for axitinib, 1.0 mg/kg/day for dasatinib, and 0.78 mg/kg/day for sunitinib. After deep anesthesia with isoflurane, the mice were decapitated, and the cerebral blood vessels were exposed to measure the maximum diameter of the basilar artery. Each drug administration condition was tested on three mice.

The distribution of the maximum diameter of the cerebral blood vessels under each drug administration condition is shown in Figure 10. The p-values in the figure were calculated using one-way ANOVA with Bonferroni correction. Since the drugs were administered from day 7 after gene mutation introduction, i.e., after the onset of cerebral artery expansion, the results in Figure 10 indicate the treatment effect on cerebral artery expansion. Compared to sunitinib, dasatinib significantly suppressed cerebral vessel expansion (p=0.040). Although there was variability, axitinib also showed a lower maximum vessel diameter overall compared to sunitinib. Thus, it was demonstrated that the treatment effect on cerebral artery expansion was highest for dasatinib, followed by axitinib, and then sunitinib at low concentration ranges.

### <Example 4: NF-κB Luciferase Reporter Assay Using Human Cells with Introduced AHNAK Mutations>

### 1. Preparation of AHNAK Expression Plasmid

Partial peptides of human AHNAK, specifically corresponding to amino acids 3801-3928 and 4649-4776 in the sequence represented by SEQ ID NO: 16 (SEQ ID NOs: 17 and 18, respectively) were amplified by PCR from cDNA derived from SH-SY-5Y cells and cloned into PCR-Blunt II TOPO (Thermo Fisher Scientific). To prepare AHNAK mutants, mutations p.G3819A, p.N3827S, and p.D4710E were introduced into *AHNAK*-TOPO using PCR-based mutagenesis with PrimeSTAR MAX DNA Polymerase (Takara). Fragments of wild-type *AHNAK* or *AHNAK* mutants were obtained by PCR from *AHNAK* wild-type-TOPO or *AHNAK* mutant-TOPO, respectively, to prepare *AHNAK* wild-type-*IRES2-eGFP* or *AHNAK* mutant-*IRES2-eGFP.* The *IRES2-eGFP* fragment was obtained by PCR from pCAG-Cre-*IRES2-GFP* plasmid (Addgene, #26646). These fragments were assembled into the pCAGGS vector (RDB08938, RIKEN BioResource Center) using NEBuilder HiFi DNA Assembly (New England BioLabs). *AHNAK-HA* was amplified by PCR using pAAV-CMV-*AHNAK-HA* plasmid as a template. *AHNAK* (BspI-NotI)-*HA* was subcloned into PCR-Blunt II TOPO. The obtained plasmid was digested with BspEI and EcoRV to remove the HA-coding fragment. This fragment was cloned into AHNAK wild-type-IRES2-eGFP or AHNAK mutant-I*RES2-eGFP* digested with BspEI-EcoRV to prepare *AHNAK* wild-type-*HA*-*IRES2*-*eGFP* (AHNAK WT) or *AHNAK* mutant*-HA-IRES2-eGFP* (AHNAK G3819A).

### 2. NF-κB Luciferase Reporter Assay

Commercially available reporter plasmids (Agilent), pNF-κB-Luciferase, and pRL-TK Luciferase (Promega) were used. HEK293 cells were seeded in 24-well plates one day before gene introduction. Each well was transfected with the prepared AHNAK expression plasmid (0.5 mg), pNF-κB-Luciferase (0.1 mg), pRL-TK-Luciferase (0.02 mg), and the reporter plasmid. For each well with wild-type AHNAK and p.G3819A mutant AHNAK, PKC inhibitor H-7 (1-(5-Isoquinolinesulfonyl)-2-methylpiperazine, 100 µM), PI3K inhibitor LY294002 (10 µM), and FGF-R inhibitor PD173074 (10 µM) were added to the medium. Wells without inhibitors served as negative controls. Each inhibitor condition was tested in four wells, and seven wells served as negative controls. One day after gene introduction, cells transfected with AHNAK expression plasmids were lysed with Glo lysis buffer (Promega), and luciferase assays were performed using the Dual-Glo Luciferase Assay System (Promega) and Infinite 200 Pro microplate reader (TECAN).

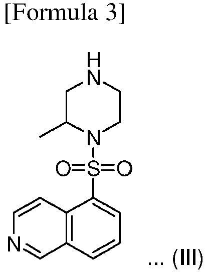

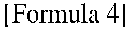

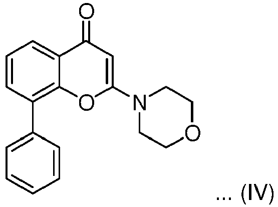

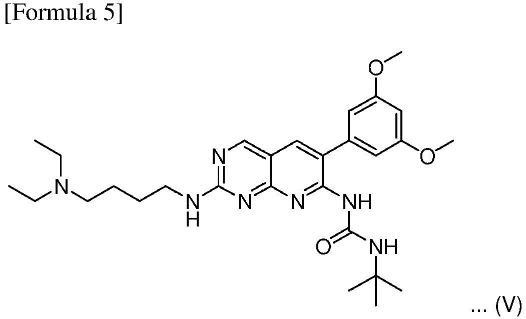

The results of the luciferase assay are shown in Figure 12. The vertical axis in the figure indicates NF-κB expression levels. It was confirmed that NF-κB in cells introduced with p.G3819A mutant AHNAK was elevated compared to wild-type AHNAK in the negative control. This elevation was also observed with p.N3827S and D4710 mutant AHNAK (data not shown). Since NF-κB is involved in inflammation, it was suggested that AHNAK mutations promote inflammatory responses in human cells. In systems with added inhibitors, there was little difference in NF-κB expression levels between wild-type and p.G3819A mutant, and overall NF-κB expression was significantly suppressed compared to the negative control. This indicates that the inflammatory response potentially promoted by AHNAK mutations may be reduced by administering PKC inhibitors, PI3K inhibitors, and FGF-R inhibitors.

### <Example 5: Study in an Animal Model with Introduced AHNAK Mutations>

To introduce AHNAK mutations into experimental animals, it is necessary to incorporate the gene into viral vectors or other vehicles. However, AHNAK is a large protein with a length of 5890 amino acids, making it difficult to incorporate its full-length gene into viral vectors. Additionally, incorporating only arbitrary fragments comprising mutations did not sufficiently express the phenotype in the introduced experimental animals. AHNAK has many repeat structures (CRU: Consensus Central Repeat Unit), and the mutation sites are also within the CRU. Therefore, we designed a low-molecular-weight version of AHNAK (AHNAKmini) that combines the C-terminal region comprising the PDZ domain, two CRUs, and the N-terminal region in this order. Figure 13 shows the structural outline of AHNAKmini. The amino acid sequence of AHNAKmini is shortened to 1943 amino acids with an N-terminal FLAG tag.

The DNA encoding the AHNAKmini with the p.G3819A mutation was chemically synthesized and subcloned directly into a pAAV vector to prepare pAAV-CMV-AHNAK (SEQ ID NO: 19). This was further modified to have the mutation corresponding to p.G3819A (specifically, changing the 3156th guanine (g) in SEQ ID NO: 19 to cytosine (c)) to prepare pAAV-CMV-AHNAK p.G3819A. HEK293T cells were transfected with the pHelper vector, pRC vector, and pAAV-CMV-AHNAK p.G3819A, and cultured, after which AAV produced by the iodixanol gradient method was harvested (AAV-CMV-AHNAK p.G3819A). As a control, AAV encoding EGFP (AAV-CMV-EGFP) was similarly prepared. After determining the serotype of AAV, it was mixed with a hydrogel of PBS comprising 15% Pluronic F-127.

Male C57BL/6N mice aged 8-11 weeks (CLEA Japan) were exposed to the middle cerebral artery according to the protocol described in A. Tamura et al., Journal of Cerebral Blood Flow and Metabolism, 1: 53-60 (1981). The hydrogel comprising AAV was applied multiple times at 5-minute intervals. The skull was covered with temporal fascia, the soft tissue was returned to its original position, and the skin was sutured. The total application amounts of AAV-CMV-EGFP and AAV-CMV-AHNAK p.G3819A were 2.4×10¹⁰ vg and 3.3×10¹¹ vg, respectively.

From day 7 post-infection (post-surgery), a solution of PD173074 dissolved in vehicle (2% DMSO, 15% PEG300) to a concentration of 0.1 mg/mL was administered at 20 µL/g/day until day 28. Similar administration was performed for a 0.02 mg/mL solution of LY294002 and a 0.02 mg/mL solution of staurosporine (PKC inhibitor, chemical formula (VI)).

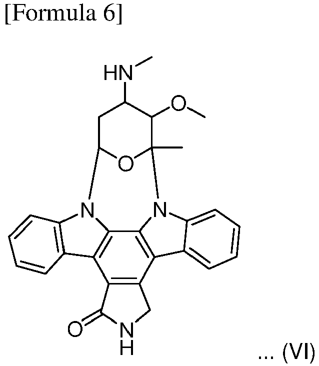

After deep anesthesia with isoflurane, the mice were decapitated and incubated at 4°C for at least 24 hours. After decapitation, the cerebral blood vessels were exposed, and the maximum diameter of the cerebral blood vessels was measured. Each drug administration condition was tested in 3 mice, with 4 mice serving as controls.

Figure 14A shows the maximum diameter of the cerebral blood vessels in the AHNAK mutation-induced cerebral aneurysm model mice with and without PD173074 administration. Error bars indicate standard error. The asterisk indicates that the p-value was less than 0.05 in the non-parametric Student's t-test with unequal variances. The group administered PD173074 showed a significant suppression of the increase in maximum cerebral vessel diameter compared to the non-administered group, suggesting that FGFR inhibitors have a treatment effect on cerebral aneurysms induced by AHNAK mutations.

Figure 14B shows the maximum diameter of the cerebral blood vessels in the AHNAK mutation-induced cerebral aneurysm model mice with and without LY294002 administration. Error bars indicate standard error. In the non-administered group, there was variability in the maximum cerebral vessel diameter, whereas in the administered group, the increase in maximum cerebral vessel diameter was suppressed in all mice. This suggests that PI3K inhibitors have a treatment effect on cerebral aneurysms induced by AHNAK mutations.

Figure 14C shows the maximum diameter of the cerebral blood vessels in the AHNAK mutation-induced cerebral aneurysm model mice with and without staurosporine administration. Error bars indicate standard error. In the non-administered group, there was variability in the maximum cerebral vessel diameter, whereas in the administered group, the increase in maximum cerebral vessel diameter was suppressed in all mice. This suggests that PKC inhibitors have a treatment effect on cerebral aneurysms induced by AHNAK mutations.

### <Example 6: Gene Expression Analysis in Tissue Samples>

The expression state of each gene was confirmed by mRNA analysis using the Visium spatial gene expression analysis system (10x Genomics) on CA tissues excised from patients. The sample preparation and gene analysis protocol were performed according to the attached documentation of the Visium system.

### 1. Preparation of CA Tissue Slides

CA tissue from two cases (FU3 and SU50) prepared as frozen sections in Reference Example 2 was used. As a control, a frozen section of normal cerebral vascular tissue prepared in the same manner was also examined under the same conditions.

One area (6.5 mm × 6.5 mm) on a Visium slide comprises 5000 spots, each fixed with thousands of DNAs with spatial barcode sequences. The sequence of the spatial barcode differs for each spot, allowing the spot location to be traced from the barcode sequence information on the Visium system.

The prepared frozen sections were set one by one in designated positions on the Visium slide. The slides were immersed in a tissue lysis buffer (10% SDS, 10 mM Trizma^{™} hydrochloride, 3.3 mg/mL proteinase (Qiagen)) pre-warmed to 50°C and incubated for 1 hour. The slides were then removed, washed with ultrapure water, and incubated twice at room temperature in a 0.08 M KOH solution (10 minutes, 5 minutes). The slides were washed with 10 mM Tris buffer (pH 7), and the surface moisture was removed to obtain tissue slides.

### 2. Staining and Imaging

The obtained tissue slides were fixed with methanol according to standard procedures and stained with H&E. The stained tissue on the slides was imaged using a fluorescence microscope system (BZ-X800, Keyence).

### 3. Permeabilization, Reverse Transcription, and cDNA Library Construction

The following operations were performed using the Visium Spatial Gene Expression Reagent Kit (10x Genomics). The slides from section 2 were set in a dedicated slide cassette and treated with permeabilization enzymes. By incubating the slides on a thermal cycler for slides at 37°C, the cells were permeabilized, and the exposed polyA mRNA was captured by the poly(dT) on the slide. A reverse transcription (RT) master mix was added to each well, and the thermal cycler was set to perform the prescribed reverse transcription reaction, forming first-strand cDNA on the slide. A cDNA library for sequencing was constructed using the first-strand cDNA as a template, according to standard methods. The cDNA had barcode sequences derived from the first strand.

### 4. cDNA Analysis

The sequences of the prepared cDNA library were determined using the Illumina HiSeq 2500 SBS V4 with 125 bp paired-end reads. Each cDNA comprised a spatial barcode sequence and mRNA sequence, allowing the spot position on the tissue section to be identified using the spatial barcode and analyzing which genes were expressed and to what extent at each spot. The Visium Spatial Gene Expression System's analysis software was used to display the expression intensity of specific genes in a two-dimensional manner on the image of the tissue section. For each case, the distribution and intensity of PDGFRβ and AHNAK gene expression on the tissue sections were analyzed.

### 5. Results

Figure 15 shows the mRNA expression status of *PDGFRβ* and *AHNAK* in frozen sections of cerebral vascular tissue from two CA patients and one control case. The areas with darker red color indicate higher mRNA expression. It was confirmed that both genes were widely overexpressed in CA tissues compared to the control. This suggested a strong involvement of these genes in cerebral aneurysms.

All publications, patents, and patent applications cited in this specification are incorporated herein by reference in their entirety.

## Claims

1. A pharmaceutical composition for treatment and/or prevention of an aneurysm comprising at least one of the following drugs as an active ingredient(s):
i) drugs targeting platelet-derived growth factor receptor β (PDGFRβ);
ii) drugs that inhibit the function of desmoyokin (AHNAK); and
iii) drugs that inhibit signaling enhanced by mutations in PDGFRβ or AHNAK.

2. The pharmaceutical composition according to claim 1, wherein the drug(s) is at least one tyrosine kinase inhibitor(s) selected from the group consisting of sunitinib, axitinib, dasatinib, gefitinib, erlotinib, lapatinib, pazopanib, vandetanib, afatinib, regorafenib, cabozantinib, osimertinib, dacomitinib, quizartinib, capmatinib, tepotinib, imatinib, sorafenib, nilotinib, crizotinib, ponatinib, ceritinib, nintedanib, lorlatinib, entrectinib, their pharmaceutically acceptable salts, solvates, and derivatives.

3. The pharmaceutical composition according to claim 2, wherein the drug(s) is at least one selected from axitinib, dasatinib, their pharmaceutically acceptable salts, solvates, and derivatives.

4. The pharmaceutical composition according to claim 1, wherein the drug(s) is the agent(s) targeting AHNAK.

5. The pharmaceutical composition according to claim 4, wherein the drug(s) is at least one selected from fibroblast growth factor receptor (FGFR) inhibitors, protein kinase C (PKC) inhibitors, and phosphatidylinositol 3-kinase (PI3K) inhibitors.

6. A method for assisting diagnosis of an aneurysm, comprising the steps:
- detecting a mutation in at least one gene of the PDGFRβ gene and AHNAK gene in a sample collected from a subject with an aneurysm; and
- predicting the therapeutic and/or preventive effect of the pharmaceutical composition according to claim 1 on the subject.

7. The method according to claim 6, wherein the PDGFRβ gene mutation corresponds to at least one mutation(s) selected from the group consisting of p.559_562del, p.563_564del, p.Y562N, p.Y562S, and p.Y562D in the amino acid sequence represented by SEQ ID NO: 2.

8. The method according to claim 6, wherein the AHNAK gene mutation is detected, and the therapeutic and/or preventive effect of FGFR inhibitors, PKC inhibitors, or PI3K inhibitors on the subject is predicted.

9. A method for determining treatment effect of a drug on an aneurysm comprising the following steps b) to d):
b) contacting human cells with the drug *in vitro*;
c) measuring the amount(s) of phosphorylated extracellular signal-regulated kinase (pERK) and/or phosphorylated tyrosine in the cells from step b); and
d) predicting the treatment effect of the drug on an aneurysm from the measurement value(s) in step c).
